(19) Europäisches Patentamt
European Patent Office
Office européen des brevets

(11) **EP 0 917 581 B1**

(12) **EUROPEAN PATENT SPECIFICATION**

(45) Date of publication and mention
of the grant of the patent:
**09.01.2008 Bulletin 2008/02**

(21) Application number: **97940030.6**

(22) Date of filing: **25.07.1997**

(51) Int Cl.:
*C12N 15/62* [(2006.01)]  *C07K 19/00* [(2006.01)]
*C12N 15/81* [(2006.01)]  *C12N 15/85* [(2006.01)]
*A01K 67/027* [(2006.01)]  *A61K 38/16* [(2006.01)]
*G01N 33/68* [(2006.01)]  *A61K 48/00* [(2006.01)]

(86) International application number:
**PCT/EP1997/004066**

(87) International publication number:
**WO 1998/004718 (05.02.1998 Gazette 1998/05)**

(54) **FUSION POLYPEPTIDES COM0PRISING AN IGE-BINDING DOMAIN AND A HSA COMPONENT, AND THEIR DIAGNOSTIC AND THERAPEUTIC USES**

FUSIONSPOLYPEPTIDE DIE EINE IGE-BINDUNGSDOMÄNE UND EINE HSA-KOMPONENTE ENTHALTEN UND DEREN DIAGNOSTISCHE UND THERAPEUTISCHE VERWENDUNG

POLYPEPTIDES DE FUSION COMPRENANT UN DOMAINE DE LIAISON IGE ET UN COMPOSANT HSA, ET LEURS UTILISATIONS DIAGNOSTIQUES ET THERAPEUTIQUES

(84) Designated Contracting States:
**AT BE CH DE DK ES FI FR GB GR IE IT LI LU NL PT SE**
Designated Extension States:
**RO SI**

(30) Priority: **26.07.1996 US 690216**

(43) Date of publication of application:
**26.05.1999 Bulletin 1999/21**

(73) Proprietors:
• **Novartis AG**
**4056 Basel (CH)**
Designated Contracting States:
**BE CH DE DK ES FI FR GB GR IE IT LI LU NL PT SE**
• **Novartis Pharma GmbH**
**1230 Wien (AT)**
Designated Contracting States:
**AT**

(72) Inventors:
• **DIGAN, Mary, Ellen**
**Morristown, NJ 07960 (US)**
• **LAKE, Philip**
**Morris Plains, NJ 07950 (US)**

• **GRAM, Hermann**
**D-79576 Weil am Rhein (DE)**

(74) Representative: **de Weerd, Petrus G.W. et al**
**Novartis International AG**
**Corporate Intellectual Property**
**4002 Basel (CH)**

(56) References cited:
**EP-A- 0 499 112**          **WO-A-89/05352**
**WO-A-90/13653**

• **R. SABAN ET AL.: "Allergens, IgE, mediators, inflammatory mechanisms." JOURNAL OF ALLERGY AND CLINICAL IMMUNOLOGY, vol. 94, no. 5, November 1994, ST. LOUIS, MO, USA, pages 836-843, XP000615476**
• **E. SCARSELLI ET AL.: "Receptor phage. Display of functional domains of the high affinity IgE receptor on the M13 phage surface." FEBS LETTERS, vol. 329, no. 1-2, 23 August 1993, AMSTERDAM, NL, pages 223-226, XP002049202**
• **U. BLANK ET AL.: "Complete structure and expression in transfected cells of high affinity IgE receptor." NATURE, vol. 337, 12 January 1989, LONDON, GB, pages 187-189, XP002049203**

**SEQUENCE LISTING**

### (1) GENERAL INFORMATION:

(i) APPLICANT:

(A) NAME: Novartis AG
(B) STREET: Schwarzwaldallee 215
(C) CITY: Basle
(E) COUNTRY: Switzerland
(F) POSTAL CODE (ZIP): CH-4058
(G) TELEPHONE: 61-324 5269
(H) TELEFAX: 61-322 7532

(ii) TITLE OF INVENTION: FUSION POLYPEPTIDES
(iii) NUMBER OF SEQUENCES: 6
(IV) COMPUTER READABLE FORM:

(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(V) CURRENT APPLICATION DATA:
APPLICATION NUMBER: WO PCT/EP97/....
(VI) PRIOR APPLICATION DATA:

(A) APPLICATION NUMBER: US 08/690216
(B) FILING DATE: 26-JUL-1996

### (2) INFORMATION FOR SEQ ID NO. 1:

(i) SEQUENCE CHARACTERISTICS:

(A) LENGTH: 257 amino acids
(B) TYPE: amino acid
(C) STRANDEDNESS: single
(D) TOPOLOGY: linear

(ii) MOLECULE TYPE: protein
(iii) HYPOTHETICAL: NO
(iii) ANTI-SENSE: NO
(v) FRAGMENT TYPE: internal
(xi) SEQUENCE DESCRIPTION: SEQ ID NO: 1:

```
Met Ala Pro Ala Met Glu Ser Pro Thr Leu Leu Cys Val Ala Leu Leu
1               5                   10                  15

Phe Phe Ala Pro Asp Gly Val Leu Ala Val Pro Gln Lys Pro Lys Val
            20                  25                  30

Ser Leu Asn Pro Pro Trp Asn Arg Ile Phe Lys Gly Glu Asn Val Thr
            35                  40                  45

Leu Thr Cys Asn Gly Asn Asn Phe Phe Glu Val Ser Ser Thr Lys Trp
        50                  55                  60

Phe His Asn Gly Ser Leu Ser Glu Glu Thr Asn Ser Ser Leu Asn Ile
65                  70                  75                  80

Val Asn Ala Lys Phe Glu Asp Ser Gly Glu Tyr Lys Cys Gln His Gln
                85                  90                  95

Gln Val Asn Glu Ser Glu Pro Val Tyr Leu Glu Val Phe Ser Asp Trp
            100                 105                 110

Leu Leu Leu Gln Ala Ser Ala Glu Val Val Met Glu Gly Gln Pro Leu
            115                 120                 125

Phe Leu Arg Cys His Gly Trp Arg Asn Trp Asp Val Tyr Lys Val Ile
    130                 135                 140

Tyr Tyr Lys Asp Gly Glu Ala Leu Lys Tyr Trp Tyr Glu Asn His Asn
145                 150                 155                 160

Ile Ser Ile Thr Asn Ala Thr Val Glu Asp Ser Gly Thr Tyr Tyr Cys
                165                 170                 175

Thr Gly Lys Val Trp Gln Leu Asp Tyr Glu Ser Glu Pro Leu Asn Ile
            180                 185                 190

Thr Val Ile Lys Ala Pro Arg Glu Lys Tyr Trp Leu Gln Phe Phe Ile
            195                 200                 205

Pro Leu Leu Val Val Ile Leu Phe Ala Val Asp Thr Gly Leu Phe Ile
    210                 215                 220

Ser Thr Gln Gln Gln Val Thr Phe Leu Leu Lys Ile Lys Arg Thr Arg
225                 230                 235                 240

Lys Gly Phe Arg Leu Leu Asn Pro His Pro Lys Pro Asn Pro Lys Asn
            245                 250                 255

Asn
257
```

(2) INFORMATION FOR SEQ ID NO. 2:

(i) SEQUENCE CHARACTERISTICS:

   (A) LENGTH: 609 amino acids
   (B) TYPE: amino acid
   (C) STRANDEDNESS: single
   (D) TOPOLOGY: linear

(ii) MOLECULE TYPE: protein
(iii) HYPOTHETICAL: NO
(iii) ANTI-SENSE: NO
(v) FRAGMENT TYPE: internal
(xi) SEQUENCE DESCRIPTION: SEQ ID NO: 2:

```
Met Lys Trp Val Thr Phe Ile Ser Leu Leu Phe Leu Phe Ser Ser Ala
1               5               10              15

Tyr Ser Arg Gly Val Phe Arg Arg Asp Ala His Lys Ser Glu Val Ala
            20              25              30

His Arg Phe Lys Asp Leu Gly Glu Glu Asn Phe Lys Ala Leu Val Leu
        35              40              45

Ile Ala Phe Ala Gln Tyr Leu Gln Gln Cys Pro Phe Glu Asp His Val
        50              55              60

Lys Leu Val Asn Glu Val Thr Glu Phe Ala Lys Thr Cys Val Ala Asp
65              70              75              80

Glu Ser Ala Glu Asn Cys Asp Lys Ser Leu His Thr Leu Phe Gly Asp
            85              90              95

Lys Leu Cys Thr Val Ala Thr Leu Arg Glu Thr Tyr Gly Glu Met Ala
            100             105             110

Asp Cys Cys Ala Lys Gln Glu Pro Glu Arg Asn Glu Cys Phe Leu Gln
        115             120             125

His Lys Asp Asp Asn Pro Asn Leu Pro Arg Leu Val Arg Pro Glu Val
        130             135             140

Asp Val Met Cys Thr Ala Phe His Asp Asn Glu Glu Thr Phe Leu Lys
145             150             155             160

Lys Tyr Leu Tyr Glu Ile Ala Arg Arg His Pro Tyr Phe Tyr Ala Pro
            165             170             175

Glu Leu Leu Phe Phe Ala Lys Arg Tyr Lys Ala Ala Phe Thr Glu Cys
            180             185             190

Cys Gln Ala Ala Asp Lys Ala Ala Cys Leu Leu Pro Lys Leu Asp Glu
        195             200             205

Leu Arg Asp Glu Gly Lys Ala Ser Ser Ala Lys Gln Arg Leu Lys Cys
        210             215             220
```

```
Ala Ser Leu Gln Lys Phe Gly Glu Arg Ala Phe Lys Ala Trp Ala Val
225             230             235             240

Ala Arg Leu Ser Gln Arg Phe Pro Lys Ala Glu Phe Ala Glu Val Ser
            245             250             255

Lys Leu Val Thr Asp Leu Thr Lys Val His Thr Glu Cys Cys His Gly
            260             265             270

Asp Leu Leu Glu Cys Ala Asp Asp Arg Ala Asp Leu Ala Lys Tyr Ile
            275             280             285

Cys Glu Asn Gln Asp Ser Ile Ser Ser Lys Leu Lys Glu Cys Cys Glu
    290             295             300

Lys Pro Leu Leu Glu Lys Ser His Cys Ile Ala Glu Val Glu Asn Asp
305             310             315             320

Glu Met Pro Ala Asp Leu Pro Ser Leu Ala Ala Asp Phe Val Glu Ser
            325             330             335

Lys Asp Val Cys Lys Asn Tyr Ala Glu Ala Lys Asp Val Phe Leu Gly
            340             345             350

Met Phe Leu Tyr Glu Tyr Ala Arg Arg His Pro Asp Tyr Ser Val Val
            355             360             365

Leu Leu Leu Arg Leu Ala Lys Thr Tyr Glu Thr Thr Leu Glu Lys Cys
    370             375             380

Cys Ala Ala Ala Asp Pro His Glu Cys Tyr Ala Lys Val Phe Asp Glu
385             390             395             400

Phe Lys Pro Leu Val Glu Glu Pro Gln Asn Leu Ile Lys Gln Asn Cys
            405             410             415

Glu Leu Phe Lys Gln Leu Gly Glu Tyr Lys Phe Gln Asn Ala Leu Leu
            420             425             430

Val Arg Tyr Thr Lys Lys Val Pro Gln Val Ser Thr Pro Thr Leu Val
            435             440             445

Glu Val Ser Arg Asn Leu Gly Lys Val Gly Ser Lys Cys Cys Lys His
    450             455             460

Pro Glu Ala Lys Arg Met Pro Cys Ala Glu Asp Tyr Leu Ser Val Val
465             470             475             480

Leu Asn Gln Leu Cys Val Leu His Glu Lys Thr Pro Val Ser Asp Arg
            485             490             495

Val Thr Lys Cys Cys Thr Glu Ser Leu Val Asn Arg Arg Pro Cys Phe
            500             505             510

Ser Ala Leu Glu Val Asp Glu Thr Tyr Val Pro Lys Glu Phe Asn Ala
            515             520             525

Glu Thr Phe Thr Phe His Ala Asp Ile Cys Thr Leu Ser Glu Lys Glu
            530             535             540

Arg Gln Ile Lys Lys Gln Thr Ala Leu Val Glu Leu Val Lys His Lys
545             550             555             560
```

```
Pro Lys Ala Thr Lys Glu Gln Leu Lys Ala Val Met Asp Asp Phe Ala
              565             570             575

Ala Phe Val Glu Lys Cys Cys Lys Ala Asp Asp Lys Glu Thr Cys Phe
          580             585             590

Ala Glu Glu Gly Lys Lys Leu Val Ala Ala Ser Gln Ala Ala Leu Gly
          595             600             605

Leu
609
```

**(2) INFORMATION FOR SEQ ID NO. 3:**

    (i) SEQUENCE CHARACTERISTICS:

        (A) LENGTH: 978 amino acids
        (B) TYPE: amino acid
        (C) STRANDEDNESS: single
        (D) TOPOLOGY: linear

    (ii) MOLECULE TYPE: protein
    (iii) HYPOTHETICAL: NO
    (iii) ANTI-SENSE: NO
    (v) FRAGMENT TYPE: internal
    (xi) SEQUENCE DESCRIPTION: SEQ ID NO: 3:

```
Met Ala Pro Ala Met Glu Ser Pro Thr Leu Leu Cys Val Ala Leu Leu
1               5               10              15

Phe Phe Ala Pro Asp Gly Val Leu Ala Val Pro Gln Lys Pro Lys Val
          20              25              30

Ser Leu Asn Pro Pro Trp Asn Arg Ile Phe Lys Gly Glu Asn Val Thr
          35              40              45

Leu Thr Cys Asn Gly Asn Asn Phe Phe Glu Val Ser Ser Thr Lys Trp
      50              55              60

Phe His Asn Gly Ser Leu Ser Glu Glu Thr Asn Ser Ser Leu Asn Ile
65              70              75              80

Val Asn Ala Lys Phe Glu Asp Ser Gly Glu Tyr Lys Cys Gln His Gln
              85              90              95

Gln Val Asn Glu Ser Glu Pro Val Tyr Leu Glu Val Phe Ser Asp Trp
          100             105             110

Leu Leu Leu Gln Ala Ser Ala Glu Val Val Met Glu Gly Gln Pro Leu
          115             120             125

Phe Leu Arg Cys His Gly Trp Arg Asn Trp Asp Val Tyr Lys Val Ile
          130             135             140

Tyr Tyr Lys Asp Gly Glu Ala Leu Lys Tyr Trp Tyr Glu Asn His Asn
145             150             155             160
```

```
Ile Ser Ile Thr Asn Ala Thr Val Glu Asp Ser Gly Thr Tyr Tyr Cys
                165             170             175

Thr Gly Lys Val Trp Gln Leu Asp Tyr Glu Ser Glu Pro Leu Asn Ile
            180             185             190

Thr Val Ile Lys Ala Pro Arg Glu Lys Tyr Trp Leu Ala Ser Gly Gly
            195             200             205

Gly Gly Ser Asp Ala His Lys Ser Glu Val Ala His Arg Phe Lys Asp
    210             215             220

Leu Gly Glu Glu Asn Phe Lys Ala Leu Val Leu Ile Ala Phe Ala Gln
225             230             235             240

Tyr Leu Gln Gln Cys Pro Phe Glu Asp His Val Lys Leu Val Asn Glu
                245             250             255

Val Thr Glu Phe Ala Lys Thr Cys Val Ala Asp Glu Ser Ala Glu Asn
            260             265             270

Cys Asp Lys Ser Leu His Thr Leu Phe Gly Asp Lys Leu Cys Thr Val
            275             280             285

Ala Thr Leu Arg Glu Thr Tyr Gly Glu Met Ala Asp Cys Cys Ala Lys
            290             295             300

Gln Glu Pro Glu Arg Asn Glu Cys Phe Leu Gln His Lys Asp Asp Asn
305             310             315             320

Pro Asn Leu Pro Arg Leu Val Arg Pro Glu Val Asp Val Met Cys Thr
                325             330             335

Ala Phe His Asp Asn Glu Glu Thr Phe Leu Lys Lys Tyr Leu Tyr Glu
            340             345             350

Ile Ala Arg Arg His Pro Tyr Phe Tyr Ala Pro Glu Leu Leu Phe Phe
            355             360             365

Ala Lys Arg Tyr Lys Ala Ala Phe Thr Glu Cys Cys Gln Ala Ala Asp
    370             375             380

Lys Ala Ala Cys Leu Leu Pro Lys Leu Asp Glu Leu Arg Asp Glu Gly
385             390             395             400

Lys Ala Ser Ser Ala Lys Gln Arg Leu Lys Cys Ala Ser Leu Gln Lys
            405             410             415

Phe Gly Glu Arg Ala Phe Lys Ala Trp Ala Val Ala Arg Leu Ser Gln
            420             425             430

Arg Phe Pro Lys Ala Glu Phe Ala Glu Val Ser Lys Leu Val Thr Asp
        435             440             445

Leu Thr Lys Val His Thr Glu Cys Cys His Gly Asp Leu Leu Glu Cys
    450             455             460

Ala Asp Asp Arg Ala Asp Leu Ala Lys Tyr Ile Cys Glu Asn Gln Asp
465             470             475             480

Ser Ile Ser Ser Lys Leu Lys Glu Cys Cys Glu Lys Pro Leu Leu Glu
            485             490             495
```

22

```
Lys Ser His Cys Ile Ala Glu Val Glu Asn Asp Glu Met Pro Ala Asp
        500                 505                 510

Leu Pro Ser Leu Ala Ala Asp Phe Val Glu Ser Lys Asp Val Cys Lys
        515                 520                 525

Asn Tyr Ala Glu Ala Lys Asp Val Phe Leu Gly Met Phe Leu Tyr Glu
    530                 535                 540

Tyr Ala Arg Arg His Pro Asp Tyr Ser Val Val Leu Leu Leu Arg Leu
545                 550                 555                 560

Ala Lys Thr Tyr Glu Thr Thr Leu Glu Lys Cys Cys Ala Ala Ala Asp
            565                 570                 575

Pro His Glu Cys Tyr Ala Lys Val Phe Asp Glu Phe Lys Pro Leu Val
        580                 585                 590

Glu Glu Pro Gln Asn Leu Ile Lys Gln Asn Cys Glu Leu Phe Lys Gln
        595                 600                 605

Leu Gly Glu Tyr Lys Phe Gln Asn Ala Leu Leu Val Arg Tyr Thr Lys
    610                 615                 620

Lys Val Pro Gln Val Ser Thr Pro Thr Leu Val Glu Val Ser Arg Asn
625                 630                 635                 640

Leu Gly Lys Val Gly Ser Lys Cys Cys Lys His Pro Glu Ala Lys Arg
            645                 650                 655

Met Pro Cys Ala Glu Asp Tyr Leu Ser Val Val Leu Asn Gln Leu Cys
        660                 665                 670

Val Leu His Glu Lys Thr Pro Val Ser Asp Arg Val Thr Lys Cys Cys
        675                 680                 685

Thr Glu Ser Leu Val Asn Arg Arg Pro Cys Phe Ser Ala Leu Glu Val
    690                 695                 700

Asp Glu Thr Tyr Val Pro Lys Glu Phe Asn Ala Glu Thr Phe Thr Phe
705                 710                 715                 720

His Ala Asp Ile Cys Thr Leu Ser Glu Lys Glu Arg Gln Ile Lys Lys
            725                 730                 735

Gln Thr Ala Leu Val Glu Leu Val Lys His Lys Pro Lys Ala Thr Lys
        740                 745                 750

Glu Gln Leu Lys Ala Val Met Asp Asp Phe Ala Ala Phe Val Glu Lys
        755                 760                 765

Cys Cys Lys Ala Asp Asp Lys Glu Thr Cys Phe Ala Glu Glu Gly Lys
    770                 775                 780

Lys Leu Val Ala Ala Ser Gln Ala Ala Leu Gly Gly Gly Gly Ser Val
785                 790                 795                 800

Pro Gln Lys Pro Lys Val Ser Leu Asn Pro Pro Trp Asn Arg Ile Phe
            805                 810                 815

Lys Gly Glu Asn Val Thr Leu Thr Cys Asn Gly Asn Asn Phe Phe Glu
            820                 825                 830
```

Val Ser Ser Thr Lys Trp Phe His Asn Gly Ser Leu Ser Glu Glu Thr
835 840 845

Asn Ser Ser Leu Asn Ile Val Asn Ala Lys Phe Glu Asp Ser Gly Glu
850 855 860

Tyr Lys Cys Gln His Gln Gln Val Asn Glu Ser Glu Pro Val Tyr Leu
865 870 875 880

Glu Val Phe Ser Asp Trp Leu Leu Leu Gln Ala Ser Ala Glu Val Val
885 890 895

Met Glu Gly Gln Pro Leu Phe Leu Arg Cys His Gly Trp Arg Asn Trp
900 905 910

Asp Val Tyr Lys Val Ile Tyr Tyr Lys Asp Gly Glu Ala Leu Lys Tyr
915 920 925

Trp Tyr Glu Asn His Asn Ile Ser Ile Thr Asn Ala Thr Val Glu Asp
930 935 940

Ser Gly Thr Tyr Tyr Cys Thr Gly Lys Val Trp Gln Leu Asp Tyr Glu
945 950 955 960

Ser Glu Pro Leu Asn Ile Thr Val Ile Lys Ala Pro Arg Glu Lys Tyr
965 970 975

Trp Leu
978

## (2) INFORMATION FOR SEQ ID NO. 4:

(i) SEQUENCE CHARACTERISTICS:

(A) LENGTH: 2955 base pairs
(B) TYPE: nucleic acid
(C) STRANDEDNESS: double
(D) TOPOLOGY: linear

(ii) MOLECULE TYPE: DNA
(iii) HYPOTHETICAL: NO
(iii) ANTI-SENSE: NO
(v) FRAGMENT TYPE: internal
(xi) SEQUENCE DESCRIPTION: SEQ ID NO: 4:

```
GAATTCACCA TGGCTCCTGC CATGGAATCC CCTACTCTAC TGTGTGTAGC CTTACTGTTC    60
TTCGCTCCAG ATGGCGTGTT AGCAGTCCCT CAGAAACCTA AGGTCTCCTT GAACCCTCCA   120
TGGAATAGAA TATTTAAAGG AGAGAATGTG ACTCTTACAT GTAATGGGAA CAATTTCTTT   180
GAAGTCAGTT CCACCAAATG GTTCCACAAT GGCAGCCTTT CAGAAGAGAC AAATTCAAGT   240
TTGAATATTG TGAATGCCAA ATTTGAAGAC AGTGGAGAAT ACAAATGTCA GCACCAACAA   300
GTTAATGAGA GTGAACCTGT GTACCTGGAA GTCTTCAGTG ACTGGCTGCT CCTTCAGGCC   360
TCTGCTGAGG TGGTGATGGA GGGCCAGCCC CTCTTCCTCA GGTGCCATGG TTGGAGGAAC   420
TGGGATGTGT ACAAGGTGAT CTATTATAAG GATGGTGAAG CTCTCAAGTA CTGGTATGAG   480
AACCACAACA TCTCCATTAC AAATGCCACA GTTGAAGACA GTGGAACCTA CTACTGTACG   540
GGCAAAGTGT GGCAGCTGGA CTATGAGTCT GAGCCCCTCA ACATTACTGT AATAAAAGCT   600
CCGCGTGAGA AGTACTGGCT TGCTAGCGGT GGAGGTGGAT CCGATGCACA CAAGAGTGAG   660
```

```
GTTGCTCATC GGTTTAAAGA TTTGGGAGAA GAAAATTTCA AAGCCTTGGT GTTGATTGCC    720
TTTGCTCAGT ATCTTCAGCA GTGTCCATTT GAAGATCATG TAAAATTAGT GAATGAAGTA    780
ACTGAATTTG CAAAAACATG TGTTGCTGAT GAGTCAGCTG AAAATTGTGA CAAATCACTT    840
CATACCCTTT TTGGAGACAA ATTATGCACA GTTGCAACTC TTCGTGAAAC CTATGGTGAA    900
ATGGCTGACT GCTGTGCAAA ACAAGAACCT GAGAGAAATG AATGCTTCTT GCAACACAAA    960
GATGACAACC CAAACCTCCC CCGATTGGTG AGACCAGAGG TTGATGTGAT GTGCACTGCT   1020
TTTCATGACA ATGAAGAGAC ATTTTTGAAA AAATACTTAT ATGAAATTGC CAGAAGACAT   1080
CCTTACTTTT ATGCCCCGGA ACTCCTTTTC TTTGCTAAAA GGTATAAAGC TGCTTTTACA   1140
GAATGTTGCC AAGCTGCTGA TAAAGCTGCC TGCCTGTTGC CAAAGCTCGA TGAACTTCGG   1200
GATGAAGGGA AGGCTTCGTC TGCCAAACAG AGACTCAAGT GTGCCAGTCT CCAAAAATTT   1260
GGAGAAAGAG CTTTCAAAGC ATGGGCAGTA GCTCGCCTGA GCCAGAGATT TCCCAAAGCT   1320
GAGTTTGCAG AAGTTTCCAA GTTAGTGACA GATCTTACCA AAGTCCACAC GGAATGCTGC   1380
CATGGAGATC TGCTTGAATG TGCTGATGAC AGGGCGGACC TTGCCAAGTA TATCTGTGAA   1440
AATCAAGATT CGATCTCCAG TAAACTGAAG GAATGCTGTG AAAAACCTCT GTTGGAAAAA   1500
TCCCACTGCA TTGCCGAAGT GGAAAATGAT GAGATGCCTG CTGACTTGCC TTCATTAGCT   1560
GCTGATTTTG TTGAAAGTAA GGATGTTTGC AAAAACTATG CTGAGGCAAA GGATGTCTTC   1620
CTGGGCATGT TTTTGTATGA ATATGCAAGA AGGCATCCTG ATTACTCTGT CGTGCTGCTG   1680
CTGAGACTTG CCAAGACATA TGAAACCACT CTAGAGAAGT GCTGTGCCGC TGCAGATCCT   1740
CATGAATGCT ATGCCAAAGT GTTCGATGAA TTTAAACCTC TTGTGGAAGA GCCTCAGAAT   1800
TTAATCAAAC AAAATTGTGA GCTTTTTAAG CAGCTTGGAG AGTACAAATT CCAGAATGCG   1860
CTATTAGTTC GTTACACCAA GAAAGTACCC CAAGTGTCAA CTCCAACTCT TGTAGAGGTC   1920
```

```
TCAAGAAACC TAGGAAAAGT GGGCAGCAAA TGTTGTAAAC ATCCTGAAGC AAAAAGAATG  1980
CCCTGTGCAG AAGACTATCT ATCCGTGGTC CTGAACCAGT TATGTGTGTT GCATGAGAAA  2040
ACGCCAGTAA GTGACAGAGT CACCAAATGC TGCACAGAAT CCTTGGTGAA CAGGCGACCA  2100
TGCTTTTCAG CTCTGGAAGT CGATGAAACA TACGTTCCCA AAGAGTTTAA TGCTGAAACA  2160
TTCACCTTCC ATGCAGATAT ATGCACACTT TCTGAGAAGG AGAGACAAAT CAAGAAACAA  2220
ACTGCACTTG TTGAGCTTGT GAAACACAAG CCCAAGGCAA CAAAAGAGCA ACTGAAAGCT  2280
GTTATGGATG ATTTCGCAGC TTTTGTAGAG AAGTGCTGCA AGGCTGACGA TAAGGAGACC  2340
TGCTTTGCCG AGGAGGGTAA AAAACTTGTT GCTGCAAGTC AAGCTGCCTT AGGTGGAGGT  2400
GGATCCGTCC CTCAGAAACC TAAGGTCTCC TTGAACCCTC CATGGAATAG AATATTTAAA  2460
GGAGAGAATG TGACTCTTAC ATGTAATGGG AACAATTTCT TTGAAGTCAG TTCCACCAAA  2520
TGGTTCCACA ATGGCAGCCT TTCAGAAGAG ACAAATTCAA GTTTGAATAT TGTGAATGCC  2580
AAATTTGAAG ACAGTGGAGA ATACAAATGT CAGCACCAAC AAGTTAATGA GAGTGAACCT  2640
GTGTACCTGG AAGTCTTCAG TGACTGGCTG CTCCTTCAGG CCTCTGCTGA GGTGGTGATG  2700
GAGGGCCAGC CCCTCTTCCT CAGGTGCCAT GGTTGGAGGA ACTGGGATGT GTACAAGGTG  2760
ATCTATTATA AGGATGGTGA AGCTCTCAAG TACTGGTATG AGAACCACAA CATCTCCATT  2820
ACAAATGCCA CAGTTGAAGA CAGTGGAACC TACTACTGTA CGGGCAAAGT GTGGCAGCTG  2880
GACTATGAGT CTGAGCCCCT CAACATTACT GTAATAAAAG CTCCGCGTGA GAAGTACTGG  2940
CTATAGTAAG AATTC    2955
```

(2) INFORMATION FOR SEQ ID NO. 5:

(i) SEQUENCE CHARACTERISTICS:

(A) LENGTH: 1827 base pairs
(B) TYPE: nucleic acid
(C) STRANDEDNESS: double
(D) TOPOLOGY: linear

(ii) MOLECULE TYPE: DNA
(iii) HYPOTHETICAL: NO
(iii) ANTI-SENSE: NO
(v) FRAGMENT TYPE: internal
(xi) SEQUENCE DESCRIPTION: SEQ ID NO: 5:

```
ATGAAGTGGG TAACCTTTAT TTCCCTTCTT TTTCTCTTTA GCTCGGCTTA TTCCAGGGGT    60
GTGTTTCGTC GAGATGCACA CAAGAGTGAG GTTGCTCATC GGTTTAAAGA TTTGGGAGAA   120
GAAAATTTCA AAGCCTTGGT GTTGATTGCC TTTGCTCAGT ATCTTCAGCA GTGTCCATTT   180
GAAGATCATG TAAAATTAGT GAATGAAGTA ACTGAATTTG CAAAAACATG TGTAGCTGAT   240
GAGTCAGCTG AAAATTGTGA CAAATCACTT CATACCCTTT TTGGAGACAA ATTATGCACA   300
GTTGCAACTC TTCGTGAAAC CTATGGTGAA ATGGCTGACT GCTGTGCAAA ACAAGAACCT   360
GAGAGAAATG AATGCTTCTT GCAACACAAA GATGACAACC CAAACCTCCC CCGATTGGTG   420
AGACCAGAGG TTGATGTGAT GTGCACTGCT TTTCATGACA ATGAAGAGAC ATTTTTGAAA   480
AAATACTTAT ATGAAATTGC CAGAAGACAT CCTTACTTTT ATGCCCCGGA ACTCCTTTTC   540
TTTGCTAAAA GGTATAAAGC TGCTTTTACA GAATGTTGCC AAGCTGCTGA TAAAGCTGCC   600
TGCCTGTTGC CAAAGCTCGA TGAACTTCGG GATGAAGGGA AGGCTTCGTC TGCCAAACAG   660
AGACTCAAAT GTGCCAGTCT CCAAAAATTT GGAGAAAGAG CTTTCAAAGC ATGGGCAGTG   720
```

```
GCTCGCCTGA GCCAGAGATT TCCCAAAGCT GAGTTTGCAG AAGTTTCCAA GTTAGTGACA   780
GATCTTACCA AAGTCCACAC GGAATGCTGC CATGGAGATC TGCTTGAATG TGCTGATGAC   840
AGGGCGGACC TTGCCAAGTA TATCTGTGAA AATCAGGATT CGATCTCCAG TAAACTGAAG   900
GAATGCTGTG AAAAACCTCT GTTGGAAAAA TCCCACTGCA TTGCCGAAGT GGAAAATGAT   960
GAGATGCCTG CTGACTTGCC TTCATTAGCT GCTGATTTTG TTGAAAGTAA GGATGTTTGC  1020
AAAAACTATG CTGAGGCAAA GGATGTCTTC CTGGGCATGT TTTTGTATGA ATATGCAAGA  1080
AGGCATCCTG ATTACTCTGT CGTGCTGCTG CTGAGACTTG CCAAGACATA TGAAACCACT  1140
CTAGAGAAGT GCTGTGCCGC TGCAGATCCT CATGAATGCT ATGCCAAGGT GTTCGATGAA  1200
TTTAAACCTC TTGTGGAAGA GCCTCAGAAT TTAATCAAAC AAAACTGTGA GCTTTTTAAG  1260
CAGCTTGGAG AGTACAAATT CCAGAATGCG CTATTAGTTC GTTACACCAA GAAAGTACCC  1320
CAAGTGTCAA CTCCAACTCT TGTAGAGGTC TCAAGAAACC TAGGAAAAGT GGGCAGCAAA  1380
TGTTGTAAAC ATCCTGAAGC AAAAAGAATG CCCTGTGCAG AAGACTATCT ATCCGTGGTC  1440
CTGAACCAGT TATGTGTGTT GCATGAGAAA ACGCCAGTAA GTGACAGAGT CACAAAATGC  1500
TGCACAGAGT CCTTGGTGAA CAGGCGACCA TGCTTTTCAG CTCTGGAAGT CGATGAAACA  1560
TACGTTCCCA AAGAGTTTAA TGCTGAAACA TTCACCTTCC ATGCAGATAT ATGCACACTT  1620
TCTGAGAAGG AGAGACAAAT CAAGAAACAA ACTGCACTTG TTGAGCTTGT GAAACACAAG  1680
CCCAAGGCAA CAAAAGAGCA ACTGAAAGCT GTTATGGATG ATTTCGCAGC TTTTGTAGAG  1740
AAGTGCTGCA AGGCTGACGA TAAGGAGACC TGCTTTGCCG AGGAGGGTAA AAAACTTGTT  1800
GCTGCAAGTC AAGCTGCCTT AGGCTTA   1827
```

**(2) INFORMATION FOR SEQ ID NO. 6:**

    (i) SEQUENCE CHARACTERISTICS:

        (A) LENGTH: 773 base pairs
        (B) TYPE: nucleic acid
        (C) STRANDEDNESS: double
        (D) TOPOLOGY: linear

    (ii) MOLECULE TYPE: DNA

(iii) HYPOTHETICAL: NO
(iii) ANTI-SENSE: NO
(v) FRAGMENT TYPE: internal
(xi) SEQUENCE DESCRIPTION: SEQ ID NO: 6:

```
ATGGCTCCTG  CCATGGAATC  CCCTACTCTA  CTGTGTGTAG  CCTTACTGTT  CTTCGCTCCA      60
GATGGCGTGT  TAGCAGTCCC  TCAGAAACCT  AAGGTCTCCT  TGAACCCTCC  ATGGAATAGA     120
ATATTTAAAG  GAGAGAATGT  GACTCTTACA  TGTAATGGGA  ACAATTTCTT  TGAAGTCAGT     180
TCCACCAAAT  GGTTCCACAA  TGGCAGCCTT  TCAGAAGAGA  CAAATTCAAG  TTTGAATATT     240
GTGAATGCCA  AATTTGAAGA  CAGTGGAGAA  TACAAATGTC  AGCACCAACA  AGTTAATGAG     300
AGTGAACCTG  TGTACCTGGA  AGTCTTCAGT  GACTGGCTGC  TCCTTCAGGC  CTCTGCTGAG     360
GTGGTGATGG  AGGGCCAGCC  CCTCTTCCTC  AGGTGCCATG  GTTGGAGGAA  CTGGGATGTG     420
TACAAGGTGA  TCTATTATAA  GGATGGTGAA  GCTCTCAAGT  ACTGGTATGA  GAACCACAAC     480
ATCTCCATTA  CAAATGCCAC  AGTTGAAGAC  AGTGGAACCT  ACTACTGTAC  GGGCAAAGTG     540
TGGCAGCTGG  ACTATGAGTC  TGAGCCCCTC  AACATTACTG  TAATAAAAGC  TCCGCGTGAG     600
AAGTACTGGC  TACAATTTTT  TATCCCATTG  TTGGTGGTGA  TTCTGTTTGC  TGTGGACACA     660
GGATTATTTA  TCTCAACTCA  GCAGCAGGTC  ACATTTCTCT  TGAAGATTAA  GAGAACCAGG     720
AAAGGCTTCA  GACTTCTGAA  CCCACATCCT  AAGCCAAACC  CCAAAAACAA  CTG     773
```

**Description**

**Field**

The invention relates to fusion polypeptides. It concerns fusion polypeptides comprising an IgE-binding domain and a human serum albumin (HSA) component and salts thereof. It also concerns polynucleotides which are intermediates in the preparation of such fusion polypeptides; appropriate recombinant expression vectors therefor, corresponding procaryotic and eucaryotic expression systems, and processes for synthesizing the fusion polypeptides.

**Background**

The interaction between immunoglobulin E (Ig**E**) and its receptors has an established role in the defense against parasitic infections in humans (M. Capron and A.Capron. Science264 [199411876-1877). In industrialized countries, however, with improved hygienic conditions, the encounter with parasites is less frequent than the disturbance of the IgE network by over-production of IgE in response to environmental allergens, resulting in allergies and other IgE- or IgE-receptor- mediated disease states.

IgE is the primary antibody involved in initiation of an immediate allergic response and is a major participant in the maintenance of the late phase response. IgE is synthesized in B lymphocytes and exerts its effects after binding to the high affinity receptor for IgE, i.e. FcεRI, which is found on the surface of allergy effector cells such as mast cells, basophils and eosinophils. IgE also exerts inducer functions through binding to its receptor on antigen presenting cells such as Langerhans cells, B cells and monocytes (G-C. Mudde et al., Allergy 50 [1995] 193-199).

An allergic response or condition is manifested when IgE molecules bind to the surface of allergy effector cells via the IgE receptor, FceRI, and become cross-linked by allergen, thereby effecting signalling to initiate degranulation of cytoplasmic granules in the cells, with accompanying release of mediators of allergy such as histamine, serotonin, prostaglandins and cytokines, and consequent local tissue edema and influx of inflammatory cells. An alternate means of stimulating allergy and associated conditions is the interaction of the IgE receptor, FcεRI, with circulating autoantibodies against FcεRI.

FcRI typically exists as a tetramer comprising an α-, a β- and two y-chains (i.e. "subunits"), although on monocytes and Langerhans cells, the β-subunit is absent.

The IgE binding site of FcεRI has been shown to be contained entirely within its α-subunit (referred to as FcεRIα) (J. Hakimi et al., J. Biol. Chem. 265 [19901 22079-22081; U. Blank et al., J. Biol Chem. 266 [1991] 2639-2646). Recombinant "knockout" mice genetically deleted for the entire α -subunit have been found to be unable to mount an allergic response to allergen challenge (D. Dombrowicz et al., Cell 75 [1993] 969-976).

FcεRIα is a heavily glycosylated polypeptide of molecular weight about 60 kD, comprising a hydrophobic transmembrane domain as well as hydrophilic extracellular ("ecto-") and cytoplasmic domains which are exposed to the outer surface of the cell. The IgE binding capability of FcεRIα has been been further localized to its extracellular portion. (J. Hakimi et al. [1990], supra: Leder et al., USP 4'962'035). It is possible to produce a soluble, secretable molecule by excising the transmembrane portion and sequences downstream therefrom (C. Ra et al., Int.Immunol. 5 [1993] 47-54); and the resulting truncation, consisting essentially of the human FcεRIα extracellular domain, has IgE-binding activity in vitro and in vivo (M. Haak-Frendscho et al., Immunol. 151 [1993] 351-358: amino acid residues 1-204 of FcεRIα fused to a truncated IgGl H chain C region; C. Ra. et al.[1993] supra: residues 1-172 of FcεRIα therein, corresponding to residues 26-197 of **SEQ. ID. NO. 1** hereof). Structural features of this fragment include two potential disulfide bridges and seven potential glycosylation sites (M. Haak-Frendscho et al. [1993], **supra).**

Therefore, truncations of FcεRIα consisting essentially of the extracellular domain can potentially be administered therapeutically to a mammal to bind serum IgE in order to prevent its binding to its high affinity receptor on allergy effector cells, and also for suppressing *de novo* IgE biosynthesis in human lymphocytes (Y. Yanagihara et al., J. Clin. Invest. 94[1994] 2162-2165).

However, effective use of an IgE-binding polypeptide such as the extracellular domain of FcεRIα for systemic treatment of IgE- or IgE receptor- mediated allergic disorders in mammals has been hindered by its extreme transience in vivo due to rapid clearance from circulating plasma. Considering that IgE- or IgE receptor- mediated diseases account for 10-20 % of physician-patient contact, an effective treatment would constitute a significant benefit to patients suffering from such conditions and an important advance in the clinical treatment of IgE- and IgE receptor- mediated disorders such as allergy and allergy-related conditions.

It would therefore be beneficial to obtain an IgE-binding polypeptide having prolonged effective serum life and thus improved clinical utility in the treatment of allergy, and in particular the systemic treatment of atopic dermatitis, atopic asthma and chronic urticaria. as well as improved activity in an efficient, cost-effective manner.

## EP 0 917 581 B1

### Summary

It has now been found that by fusing an IgE-binding domain to a human serum albumin (HSA) component shown in Seq ID 3, the resulting fusion polypeptide has extended serum half-life relative to the IgE-binding domain alone, without loss of IgE-binding activity. resulting in IgE-binding polypeptides indicated for use in the systemic treatment of allergy and otherIgE-mediated disorders. Systemically administered IgE-binding polypeptide will bind to serum IgE as well as to circulating auto-antibodies against the IgE receptor, FcεRIα, preventing them from binding to cell-bound-FcRIot, and thus preventing and/or inhibiting an allergic reaction and its associated manifestations.

It has further been found that significant improvements in IgE-binding activity can be obtained by using fusion polypeptides of the invention defined in the claims comprising more than one IgE-binding domain per molecule. For example, a "dimer" molecule of the invention has been found to have significantly increased IgE-binding activity relative to a "monomer" of the invention.

The term "dimer" herein refers to a fusion polypeptide of the invention possessing two IgE-binding domains. It has further been found that a dimer molecule of the invention possesses unexpectedly favorable activity.

The invention therefore is directed to fusion polypeptides and salts thereof of SEQ.ID.NO 3 or of residues $Val_{26}$-$Leu_{978}$ of SEQ.ID.NO.3.

The invention is further directed to polynucleotide intermediates therefor.

The invention is further directed to a method for the prevention and/or treatment of IgE- or 1gE-receptor-mediated disorders, and in particular, allergic reactions such as atopic dermatitis, atopic asthma and chronic urticaria, comprising the administration of the fusion polypeptides of the invention or pharmaceutically acceptable salts thereof to a subject in need of such treatment; and to pharmaceutical compositions comprising the fusion polypeptides of the invention or pharmaceutically acceptable salts thereof, together with a least one pharmaceutically acceptable carrier or diluent.

The invention is further directed to polynucleotides which are intermediates in the preparation of the fusion polypeptides, to oligonucleotide intermediates to be used to construct them; to the peptides encoded by such oligonucleotides; to recombinant vectors for expressing the fusion molecules; to procaryotic or eucaryotic (especially, mammalian) expression systems; and to processes for preparing the fusion polypeptides or physiologically functional equivalents thereof using such expression systems.

### Description of SEQ. ID. NOs. and related definitions

**SEQ. ID. NO. 1**: Amino acid sequence of dominant form of full length native human FcεRIα, including signal sequence.
The term **"pre-IgE$^R$"** refers to residues $Met_1$-$Leu_{204}$ of SEQ. ID. NO. 1. The term **"IgE$^R$"** refers to the mature form of pre-IgE$^R$ and constitutes residues $Val_{26}$-$Leu_{204}$ of SEQ. ID. NO. 1 (i.e. the extracellular domain of FcεRIα).

**SEQ. ID. NO. 2:** Amino acid sequence of dominant form of native prepro-HSA (referred to herein as **"prepro HSA I"),** comprising residues $Met_1$-$Leu_{609}$.
The dominant form of the mature native protein (referred to herein as **"HSA I"**) is represented by residues $Asp_{25}$-$Leu_{609}$ of SEQ. ID. NO. 2.
The term **"prepro-HSA II"** represents a truncation of the native sequence by one amino acid ($Leu_{609}$) at the carboxy terminus, and therefore refers to residues $Met_1$-$Gly_{608}$ of SEQ. ID. NO. 2.
The mature form of prepro-HSA II, referred to herein as **"HSA II",** is represented by residues $Asp_{25}$-$Gly_{608}$ of SEQ. ID. NO. 2.

**SEQ. ID. NO. 3:** Amino acid sequence encoded by the EcoRI fragment of plasmid R-H-R/SK #50 prepared in **Example 5,** comprising: "pre-IgE$^R$" sequence at residues 1-204; linker $AlaSer(Gly)_4Ser$ (referred to hereinafter as **"L$_1$"**) at residues 205-211; HSA II sequence at residues 212-795: linker $(Gly)_3Ser$ (referred to hereinafter as **"L$_2$"**) at residues 796-799; and the "IgE$^R$" sequence at residues 800-978.
A mature dimeric fusion polypeptide of the invention, referred to herein as **"IgE$^R$ - L$_1$- HSA II - L$_2$ - IGE$^R$"** or, alternatively, as **"IgE$^R$ - HSA - IgE$^R$ Dimer",** expressed from CHO cells in the manner described in **Example 7,** has the amino acid sequence $Val_{26}$-$Leu_{978}$ of SEQ. ID. NO. 3.

**SEQ. ID. NO. 4:** Nucleotide sequence of the EcoRI fragment of plasmid R-H-R/SK #50 of **Example 5**. comprising: a polynucleotide sequence encoding "pre-IgE$^R$" at positions 10-621; an oligonucleotide encoding L$_1$ at positions 622-642; a polynucleotide encoding HSA II at positions 643-2394; an oligonucleotide encoding L$_2$ at positions

2395-2406; and a polynucleotide encoding "IgE$^R$" at positions 2407-2943; with 2 stop codons at positions 2944-2949. Restriction sites at the ends of the coding fragments and in the linker regions are at positions 1-6; 622-627; 637-642: 2387-2393; 2401-2406; and 2950-2955. A Kozak sequence is at nucleotide positions 7-9.

Point mutations differing from the consensus HSA nucleotide sequence are at positions 804, 1239; 1290; 1446, 1815, 2064 and 2079. Because the point mutations are in the wobble position, they do not affect the amino acid sequence.

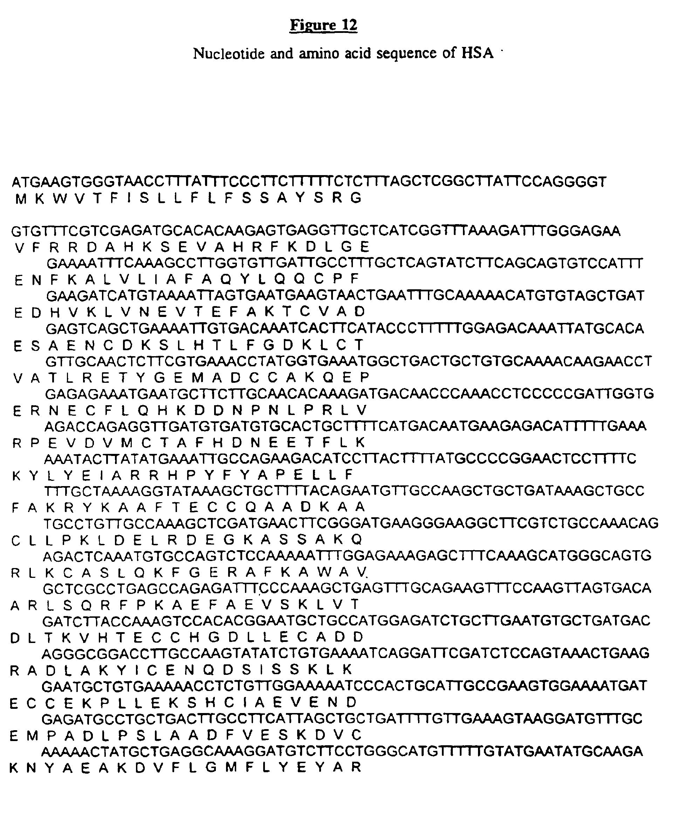

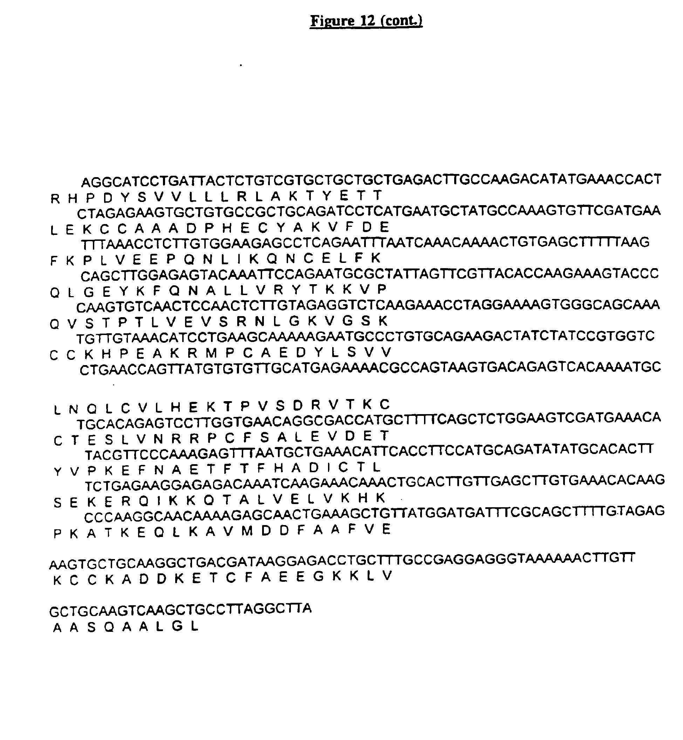

**Seq. ID. NO. 5:** Nucleotide sequence of the dominant form of native prepro-HSA corresponding to FIG. 12 and to the amino acid sequence of SEQ. ID-NO.2.

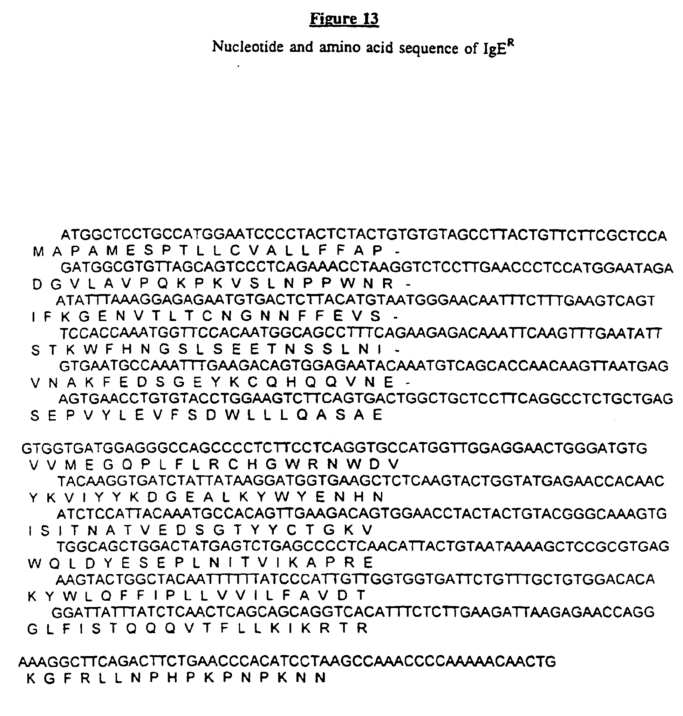

**Seq. ID. NO. 6**: Nucleotide sequence of the dominant form of full length native human FcεRIα, including signal sequence, corresponding to FIG. 13 and to the amino acid sequence of SEQ.ID.NO. 1.

## Description of the Figures

In the following Figures, the indicated molecules are read directionally, i.e. the left side corresponding to the amino (or 5'-) terminus and the right side corresponding to the carboxy (or 3'-) terminus.

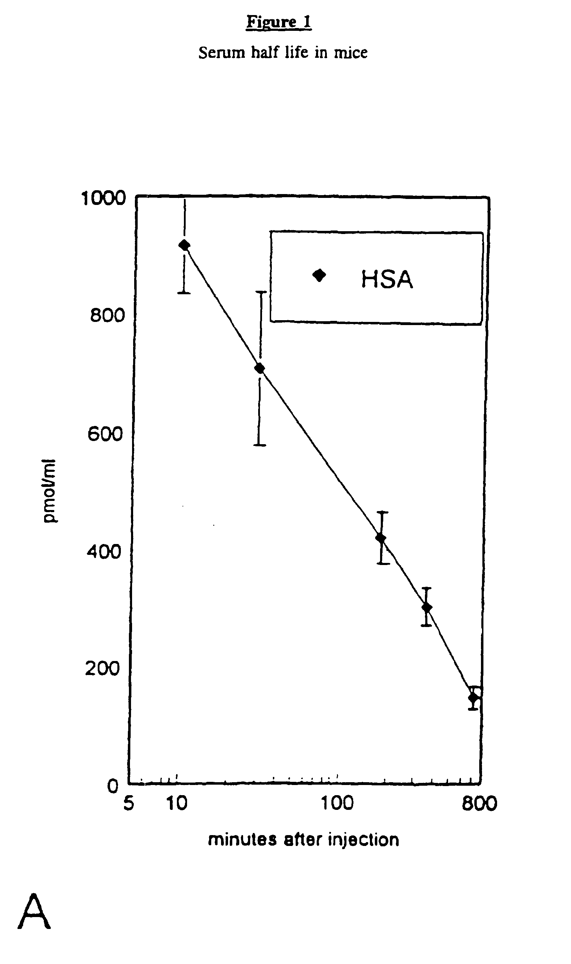

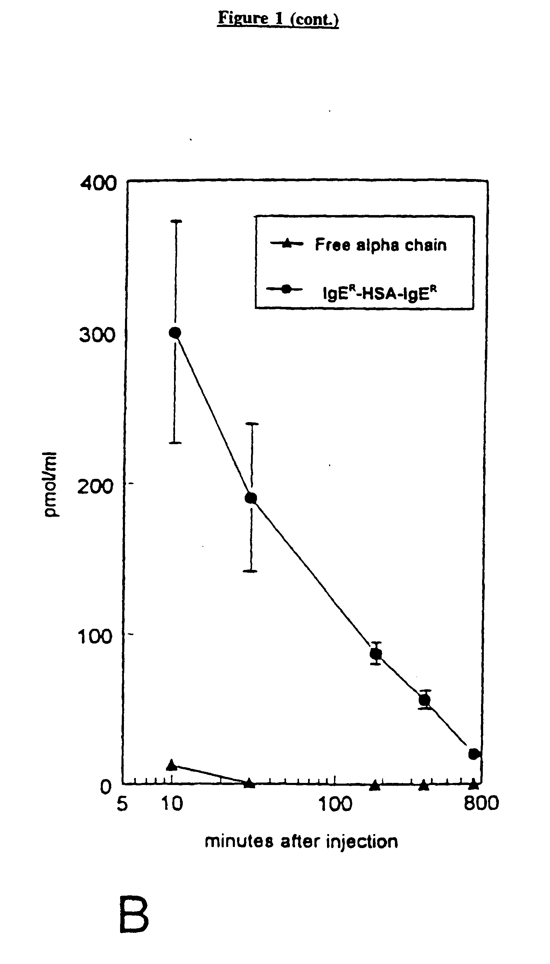

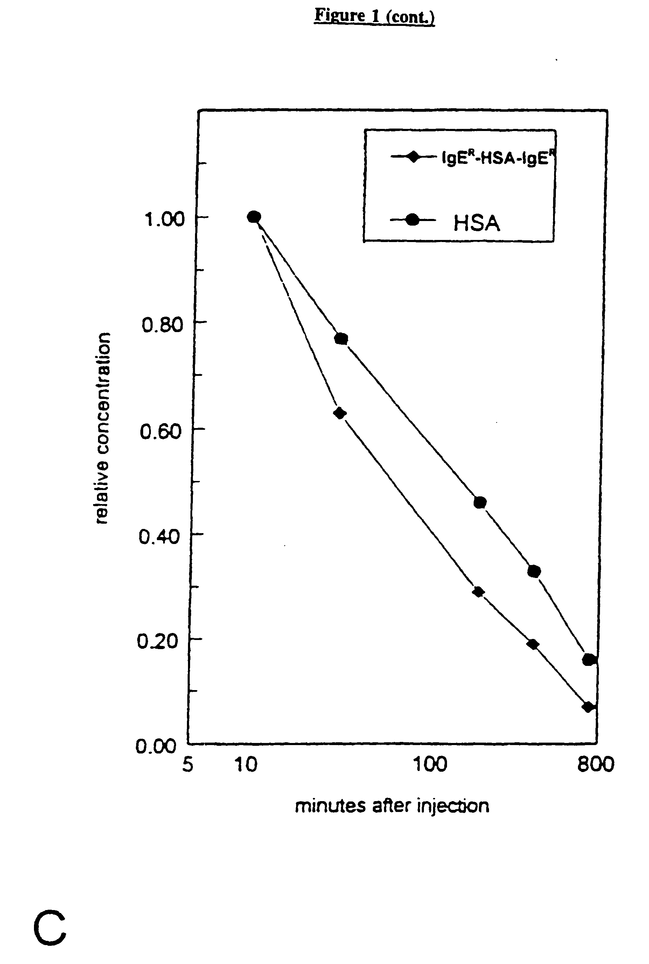

**FIGS. 1A-C:** Serum half life in mice: Protein concentration in vivo (in picomoles of protein per ml of serum) over time (10-800 minutes after injection) of

(A) free HSA I protein, and
(B) IgE$^R$ protein (referred to as "Free alpha chain") and IgE$^R$ - L$_1$ - HSA II - L$_2$ - IgE$^R$ dimeric fusion polypeptide (referred to as "IgE$^R$ - HSA - IgE$^R$" dimer) prepared from CHO cells as described in **Example 7.**
(C) Serum half life of free HSA I from (A) compared with that of dimeric fusion polypeptide ("IgE$^R$ - HSA - IgE$^R$") from (B) by normalizing to 1 with respect to serum concentrations at 10 minutes after injection.

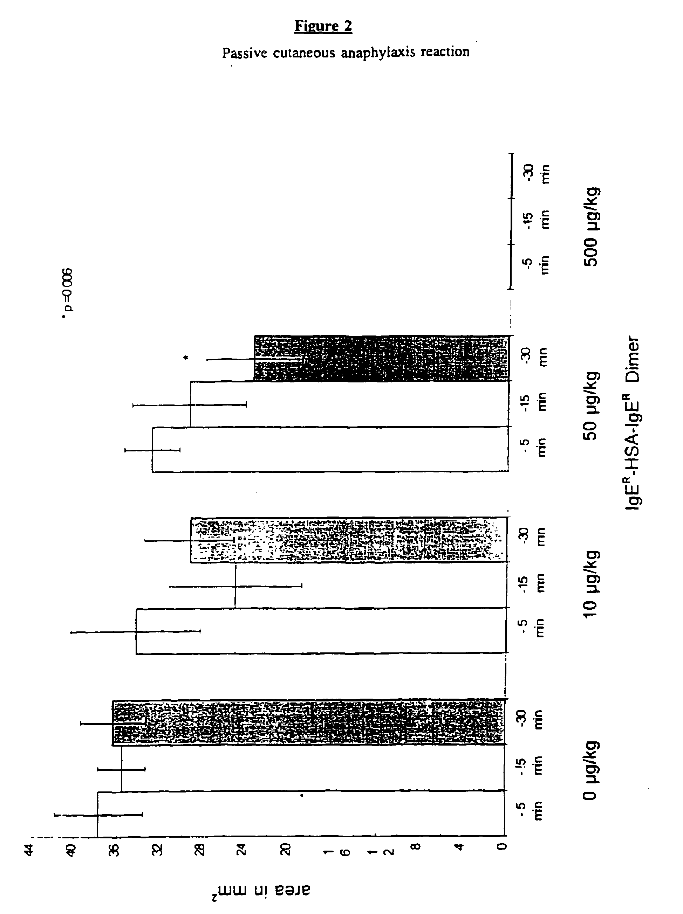

**FIG. 2:** Extravasation resulting from passive cutaneous anaphylaxis reaction
in mice administered intravenous serial dilutions (10 μg/kg, 50 μg/kg or 500 μg/kg) of IgE$^R$ - L$_1$- HSA II - L$_2$ - IgE$^R$ polypeptide ("IgE$^R$ - HSA - IgE$^R$ Dimer") prepared as described in Example 7 (the control group receiving 0 μg/kg); area in mm$^2$; at intervals of 5, 15 or 30 minutes prior to sensitization by intradermal injection of monoclonal mouse IgE anti-dinitrophenyl (DNP) antibody and subsequent challenge with DNP-bovine serum albumin solution containing 1% Evans blue.

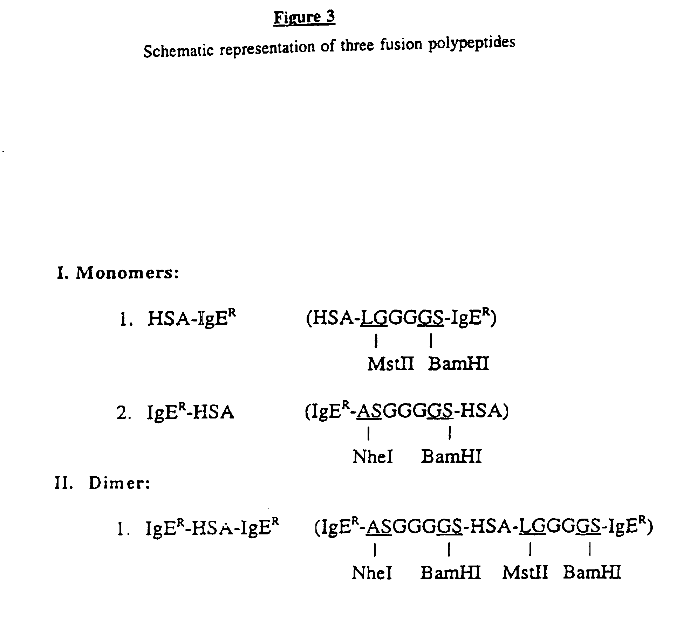

**FIG. 3**: Schematic representation of three fusion polypeptides of the invention (two monomers and one dimer), with the polypeptide linkers also shown:

I. Monomers:

(1.) HSA-leading monomer comprising HSA II (referred to in the Figure as "HSA") fused via L$_2$ ("GGGS") to IgE$^R$. The nucleotides encoding positions Leu$_{607}$Gly$_{608}$ of HSA II contain a unique MstII site as indicated, and the nucleotides encoding GlySer of L$_2$ contain a BamHI site (encoded amino acids underlined);
(2.) IgE-binding domain-leading monomer comprising IgE$^R$ fused at its carboxy terminus via L$_1$ (i.e. "AS-GGGGS") to HSA II (referred to in the Figure as "HSA"). The oligonucleotide encoding L$_1$ contains a NheI site and a BamHI site respectively (encoded amino acids AlaSer and GlySer of L$_1$ are underlined).

II. Dimer: IgE-binding domain-leading dimer comprising a first IgE$^R$ fused at its carboxy terminus via L$_1$ to the amino terminus of HSA II (referred to in the Figure as "HSA"), the carboxy terminus of which is fused to a second IgE$^R$ via L$_2$ with restriction sites in the encoding polynucleotide as described above for the monomer.

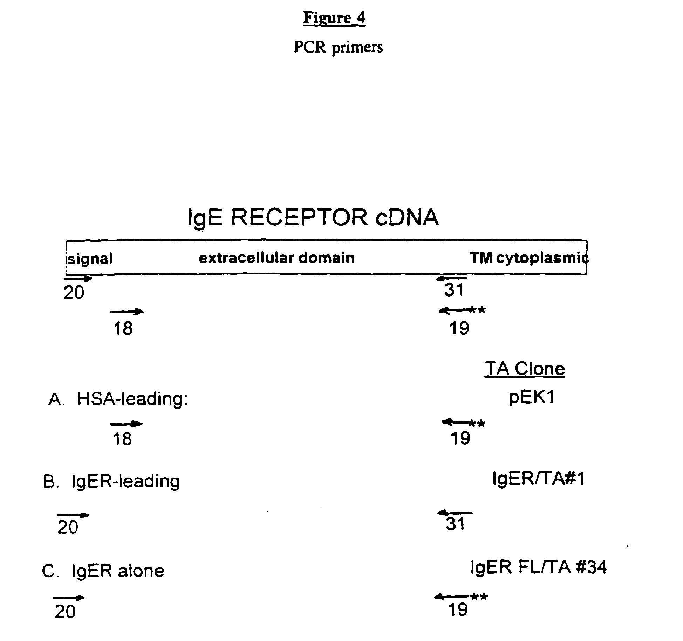

**FIG. 4:** PCR primers to truncate full length human FcεRIα cDNA (referred to as "IgE Receptor cDNA") to obtain DNA encoding:

(i) IgE$^R$
[BamHI site added to 5' end of coding strand for FcεRIα by oligonucleotide #18; and stop codon and EcoRI and SalI sites added to 3' end of non-coding strand by oligonucleotide #19];
(ii) pre-IgE$^R$
[oligonucleotide #20 adding SstI, EcoRI, and Kozak sites to 5' end of coding strand for FcεR1α; oligonucleotide #31 adding NotI and NheI (and deleting a second NheI site) in non-coding strand for FcεRIα];
(iii) pre-IgE$^R$
[oligonucleotides #20 and #19 used as described above]:

(A.) "HSA-leading": subcloning of (i) above into SK vector, providing cloning vector TA clone pEK1 used in construction of HSA II - leading monomer;
(B.) "IgER-leading": subcloning of (ii) into SK vector, providing construct IgER/TA#1 used to prepare IgE$^R$-leading monomer;
(C.) "IgER alone": subcloning of (iii) into SK vector, providing construct IgER FL/TA#34 used in expression of mature IgE$^R$ for use as a standard.
Double asterisks represent two stop codons.

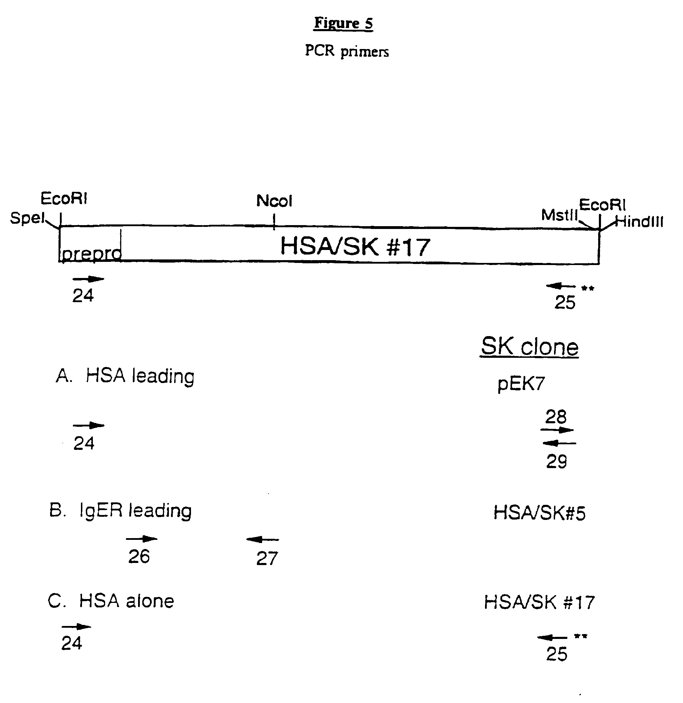

FIG. 5: PCR primer pairs to truncate full length human prepro-HSA I cDNA to yield cDNA encoding:

(i) prepro-HSA 11 fused at carboxy terminus to L$_2$
[oligonucleotide # 24 adding SpeI, EcoRI and Kozak sequence to 5'-end of coding strand; oligonucleotides #28 and #29 adding a linker encoding MstII and HindIII sites at the
3' terminus of HSA II):
(ii) L$_1$ fused to 5' terminus of HSA II
[oligonucleotide #26 adding NotI. NheI. linker and BamHI sites to coding strand; oligonucleotide #27 containing NcoI site in non-coding strand];
(iii) prepro-HSA I
[oligonucleotide #24 used as above; and oligonucleotide #25 adding stop, EcoRI and HindIII sites to 3' end of non-coding strand]:

(A.) "HSA leading": subcloning of (i) into SK vector, providing pEK7 used to construct HSA II - leading monomer:
(B.) "IgER leading": subcloning of (ii) into SK vector, providing HSA/SK#5 used to construct IgE$^R$-leading monomer;
(C.) "HSA alone": subcloning of (iii) into SK vector, providing HSA/SK#17 encoding mature native human serum albumin protein (i.e. HSA I) for use as a standard.

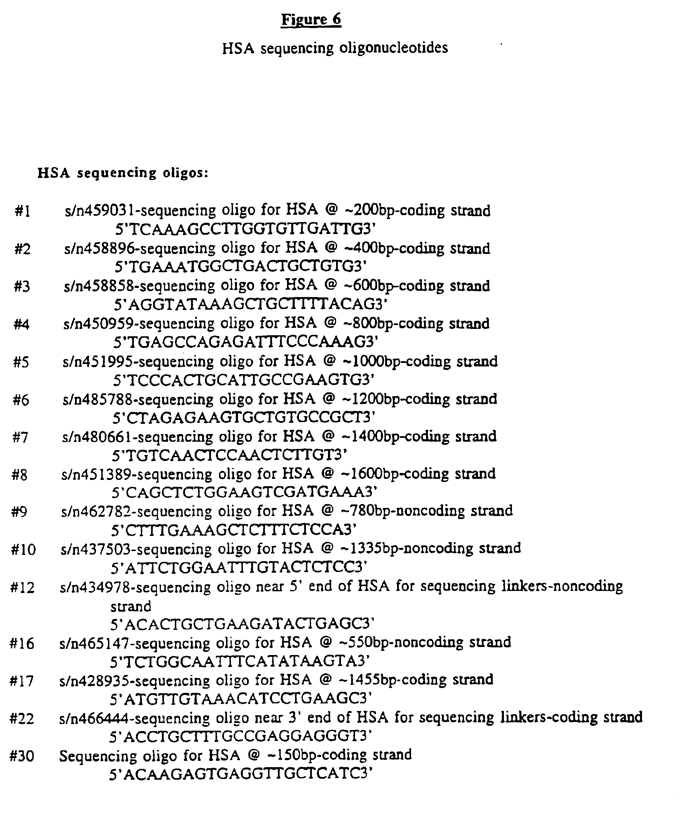

FIG. 6: Human serum albumin (HSA) sequencing oligonucleotides #1-10, 12, 16, 17, 22 and 30, used to sequence materials cloned in TA and after linkage to IgE-binding fragments.

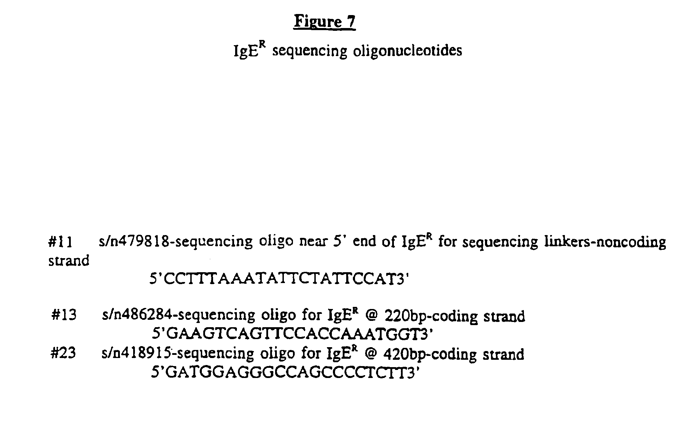

FIG. 7: IgE receptor sequencing oligonucleotides #11, 13 and 23, used to sequence fragments cloned in TA and after linkage to HSA component.

FIG. 8:

(A) Mutagenic oligonucleotides #14 and #15 for human serum albumin;
(B) PCR and linker oligonucleotides #18-20, 24-29 and 31 for constructing fusion polypeptides.

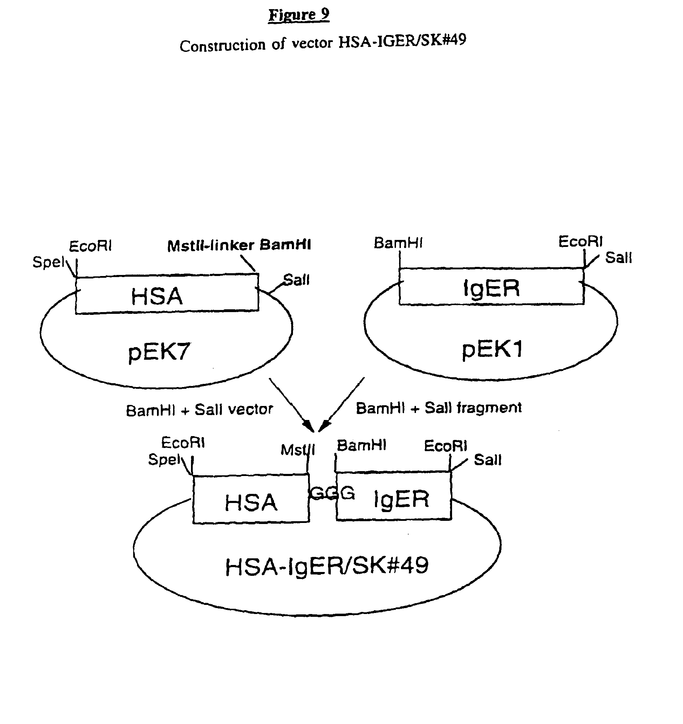

FIG. 9: Construction of vector HSA-IgER/SK#49, comprising a polynucleotide encoding prepro-HSA II (referred to in the Figure as "HSA") fused at 3'-terminus via oligonucleotide encoding linker L$_2$ (represented in FIG.9 by "GGG") to 5'-terminus of polynucleotide encoding IgE$^R$ (referred to in the Figure as "IgER"), by ligating the BamHI, SalI fragment of pEK1 into BamHI.SalI-cut pEK7.

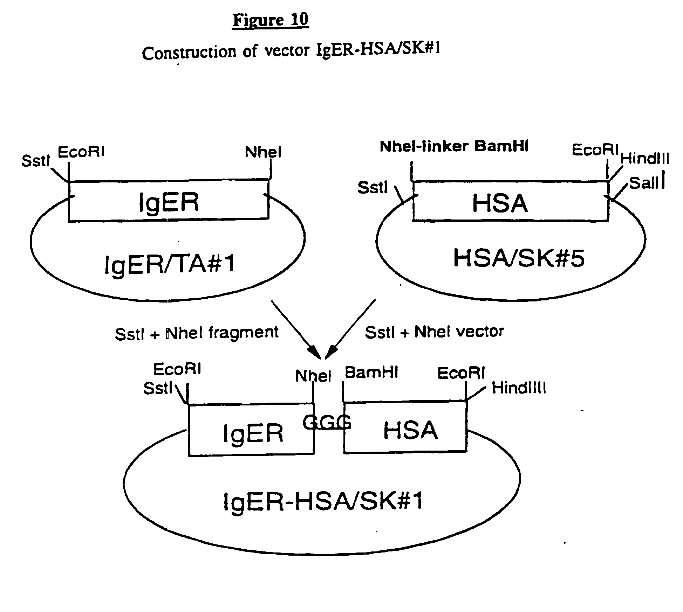

FIG. 10: Construction of vector IgER-HSA/SK#1, comprising a polynucleotide encoding pre-IgE$^R$ (referred to in the Figure as "IgER") fused at 3'-terminus via oligonucleotide for linker L$_1$ (represented by "GGG") to 5'-terminus of polynucleotide encoding HSA II ("HSA"), by ligating the SstI, NheI fragment of IgER/TA#I into SstI,NheI - cut HSA/SK#5.

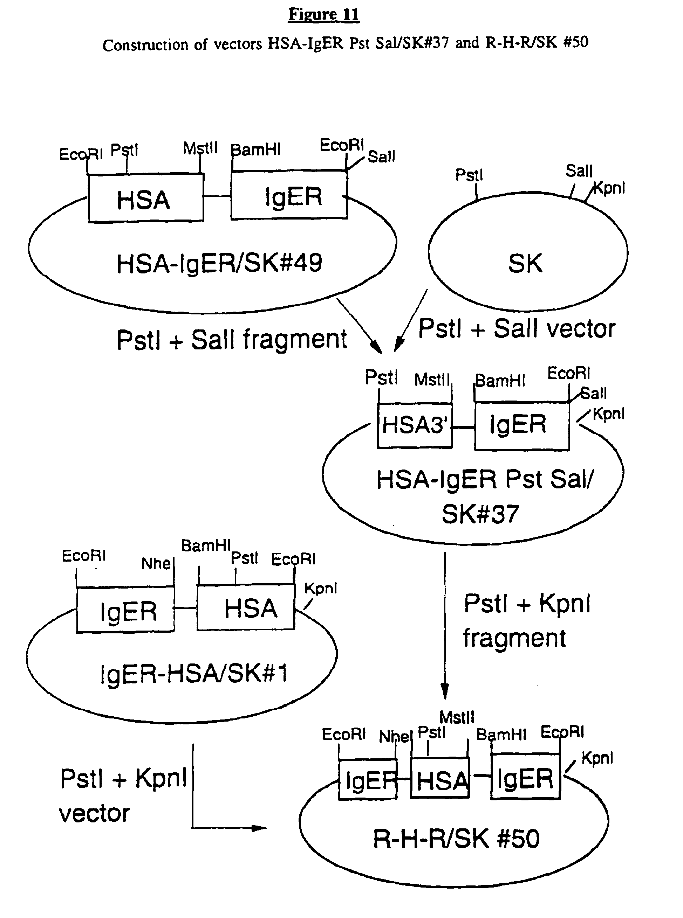

FIG. 11: Construction of vector HSA-IgER Pst Sal/SK#37, comprising a polynucleotide encoding HSA II (indicated by "HSA3") which is fused at the 3' terminus via oligonucleotide for L$_2$ (not depicted) to 5'-terminus of polynucleotide encoding IgE$^R$ (indicated as "IgER"), by ligating the PstI, SalI fragment of HSA-IgER/SK#49 into SK vector. Also shown is construction of a vector R-H-R/SK #50 comprising a polynucleotide encoding pre-IgE$^R$ (referred to as "IgER") fused at its 3' terminus via oligo for L$_1$ (not depicted) to 5'-terminus of polynucleotide encoding HSA II ("HSA"), which in turn is fused via oligonucleotide for $L_2$ (not depicted) to a polynucleotide encoding $IgE^R$ ("IgER"). The vector is prepared by ligating the PstI,KpnI fragment of HSA-IgER Pst Sal/SK#37 into PstI.KpnI - cut IgER-HSA/SK#1.

**FIG. 12:** Nucleotide sequence of human serum albumin and amino acid sequence corresponding to **SEQ. ID. NO. 2** and **SEQ. ID. NO. 5.**

**FIG. 13:** Nucleotide sequence of $IgE^R$ and amino acid sequence corresponding to **SEQ. ID. NO. 1** and **SEQ. ID. NO. 6.**

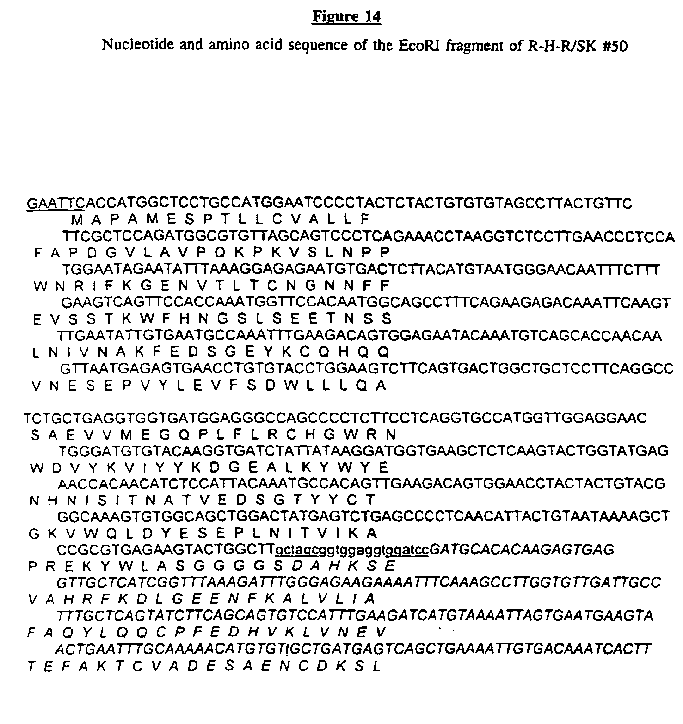

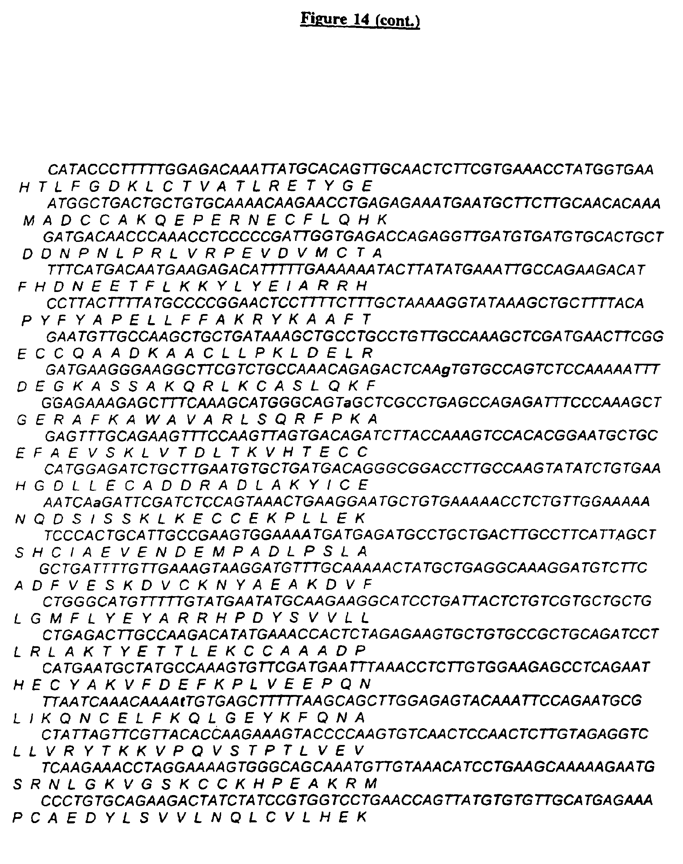

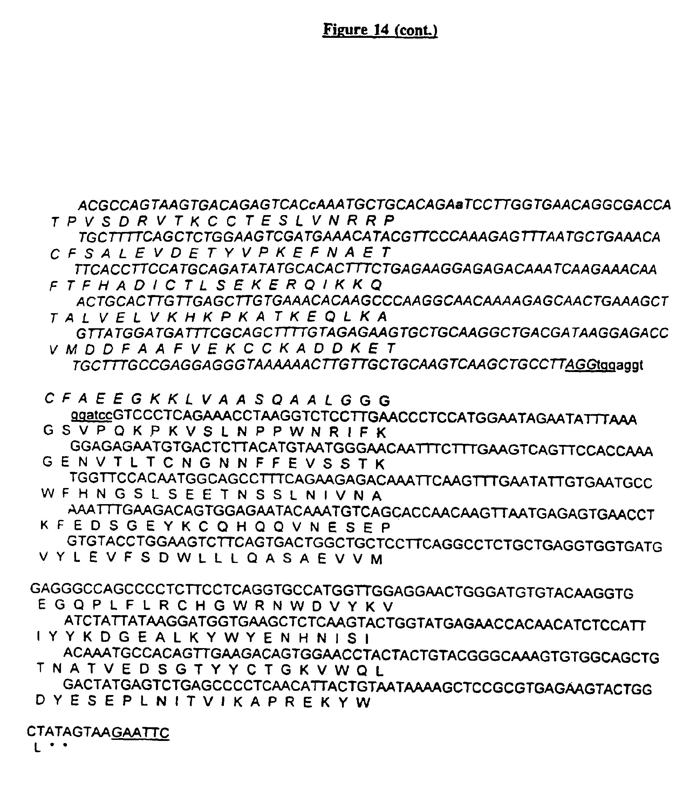

**FIG. 14**: Nucleotide and amino acid sequences of the EcoRI fragment of R-H-R/SK #50 corresponding to SEQ. **ID. NO. 3** and **SEQ. ID. NO. 4,** encoding the R1 - HSA - R1 dimeric fusion protein. The HSA sequence is shown in italics- Linker sequences are shown in lower case. Point mutations differing from the consensus HSA nucleic acid sequence are shown in bold lower case. Because the point mutations are in the wobble position, they do not affect the amino acids sequence. Restriction sites at the ends of the fragments and in the linker region are underlined.

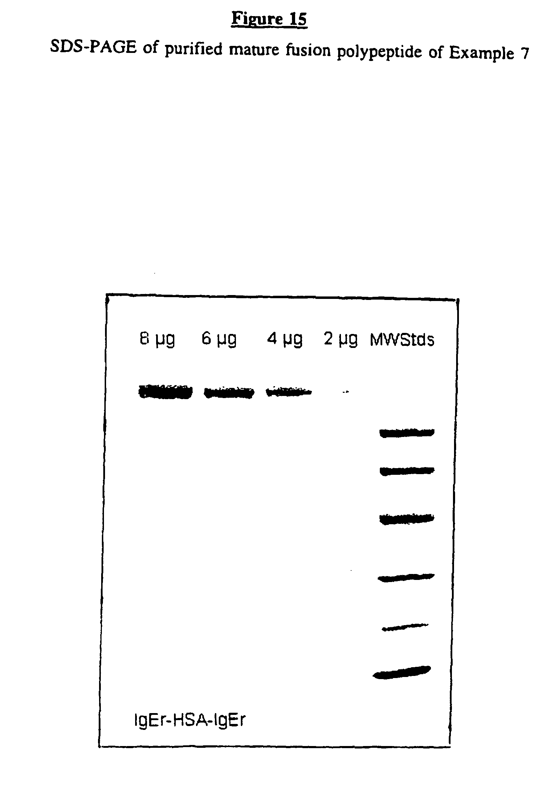

**FIG. 15 :** SDS-PAGE of purified mature fusion polypeptide of Example 7:
Total amounts applied to gel: 8 $\mu$g (lane 1), 6 $\mu$g (lane 2), 4 $\mu$g (lane 3) and 2 $\mu$g (lane 4); molecular weight standards: 97.4. 66.2. 45. 31, 21.5 and 14.4 kDa (lane 5).

## Detailed description

The invention concerns fusion polypeptides and salts thereof of SEQ.ID.NO 3 or of residues $Val_{26}$-$Leu_{978}$ of SEQ.ID.NO 3.

The cDNA and deduced amino acid sequence of human $Fc\varepsilon RI\alpha$ are known (J.Kochan et al., Nucleic Acids Research 16 [1988] 3584; and Leder et al., USP 4'962'035). Human $Fc\varepsilon RI\alpha$ encodes an $NH_2$-terminal signal peptide [amino acid residues (including the first Met) $Met_1$-$Ala_{25}$], two immunoglobulin-like extracellular domains (residues $Val_{26}$-$Leu_{204}$), a hydrophobic transmembrane region($Gln_{205}$-$Ile_{224}$), and a hydrophilic cytoplasmic tail (residues $Ser_{225}$-$Asn_{257}$) (with reference to SEQ. ID. NO.1). The signal peptide is cleaved during intracellular processing. The full length amino acid sequence of the dominant form of native humanFc#RIa is included herein as **SEQ. ID. NO. 1.**
"$IgE^R$" herein refers to amino acid sequence $Val_{26}$-$Leu_{204}$ of SEQ. ID. NO. 1 (encoding the extracellular domain); and the term "pre-$IgE^R$" refers to residues $Met_1$-$Leuz_{204}$ of SEQ. ID. NO. 1 (encoding the signal sequence upstream of the extracellular domain).
The other major constituent of the recombinant fusions of the invention, human serum albumin(HSA), constitutes the most abundant plasma protein, contributing 60 % on a per weight basis of the total protein content of plasma. A molecule of human serum albumin consists of a single non-glycosylated polypeptide chain of 5 85 amino acids of molecular weight 66.5 kDa. A feature specific to albumin is its complex disulfide bond pattern (F.F. Clerc et al., J.Chromatogr. 662 [1994] 245-259). Human serum albumin is widely distributed throughout the body, in particular in the intestinal and blood compartments where it is mainly involved, as the most abundant protein of the serum, in the maintenance of osmolarity and plasma volume. Furthermore, it is slowly cleared by the liver and displays an in vivo half-life of 14-20 days in humans (T.A. Waldmann, "Albumin Structure. Function and Uses", Pergamon Press [1977] 255-275). Human serum albumin is devoid of enzymatic or immunological function. It is a natural carrier involved in the endogenous transport and delivery of various natural as well as therapeutic molecules (H. Lu et al., FEBS Lett. 356 [1994]56-59; WO 93/15199; EP 648499; P. Yeh et al., PNAS USA 89 [1992] 1904-1908; EP 413622).
Human cells synthesize serum albumin initially in the form of a prepro- polypeptide. A signal sequence of 18 amino acids (including the first Met) is removed when the protein passes through the lumen of the endoplasmic reticulum, leaving still 6 amino acids at the N-terminus (in the predominant form: Arg-Gly-Val-Phe-Arg-Arg) which are then subject to proteolytic excision during or immediately following transport through the secretory apparatus.
Human serum albumin is well-known to be polymorphic (D.C. Carter and JX. Ho, Adv. Prot. Chem. 45 [1994] 153-203). For example, albumin Naskapi has $Lys_{372}$ in place of $Glut_{372}$, and proalbumin Christchurch has an altered pro- sequence, i.e. $Glu_{24}$ instead of $Arg_{24}$ (Latta et al. USP 5'100'784).
The complete amino acid sequence of the dominant form of naturally occurring protein, referred to herein as "prepro-HSA I", is known (A. Dugaiczyk et al., PNAS USA 79[1982]71-75) **(SEQ. ID. NO. 2).** The dominant form of the mature protein("HSA I") is represented by $Asp_{25}$-$Leu_{609}$ of SEQ. ID. NO. 2.
Especially preferred is the fusion polypeptide of Example 7, especially without leader sequence, i.e. the polypeptide $Val_{26}$-$Leu_{978}$ of SEQ-ID.NO.3.
Any peptide linker (expressed as "L" in formulaeI-V herein) preferably allows independent folding and activity of the IgE-binding domain; is free of a propensity for developing an ordered secondary structure which could interfere with the IgE-binding domain or cause an immunological reaction in the patient, and has minimal hydrophobic or charge characteristics which could interact with the IgE-binding domain.

Linkers of the present invention include: GlyGlyGlySer ("$L_1$" herein) and AlaSerGlyGlyGlyGlySer ("$L_2$" herein).

The invention also comprises polynucleotides which are intermediates in the preparation of the recombinant fusion polypeptides of the invention.

As a further aspect, there is provided a process for preparing a recombinant fusion polypeptide as defined above or salt thereof, which comprises:

(a) transforming a host cell with a vector comprising DNA encoding a fusion polypeptide as defined above;

(b) expressing the fusion polypeptide in that cell to yield a fusion polypeptide as defined above; and

(c) recovering the resultant polypeptide from the host cell, preferably as a secreted product, optionally in the form of a salt thereof.

A still further aspect of the present invention provides vectors, preferably plasmids, for use in the expression of the fusion polypeptides. These vectors comprise DNA encoding the polynucleotides defined above. In general, appropriate vectors which can transform microorganisms capable of expressing the fusion polypeptides include expression vectors comprising nucleotide sequences coding for the fusion polypeptides joined to transcriptional and translational regulatory sequences, such as promoters, which together constitute an expression cassette. The promoters may derive from genes of the particular host used, or such control regions may be modified, for example, by in vitro site-directed mutagenesis, by introduction of additional control elements or synthetic sequences. The expression cassette specifically used in the present invention thus also includes a transcription and translation termination region which is functional in the intended host and which is positioned at the 3' end of the sequence encoding the hybrid macromolecule. In addition to the expression cassette, the vector will include one or several markers enabling the transformed host to be selected. Such markers include markers conferring resistance to antibiotics such as G418. These resistance genes will be placed under the control of the appropriate transcription and translation signals allowing for expression in a given host.

More particularly, the preparation of recombinant fusion polypeptides of the invention may be effected e.g. as follows:

A. Construction of fusion protein expression vectors

The first step in the construction of recombinant fusion polypeptides is to subclone portions of the fusion polypeptides in cloning vectors. In this context, a "cloning vector" is a DNA molecule, such as a plasmid, cosmid or bacteriophage, that can replicate autonomously in a host prokaryotic cell. Cloning vectors typically contain one or a small number of restriction endonuclease recognition sites at which foreign DNA sequences can be inserted in a determinable fashion without loss of an essential biological function of the vector, as well as a marker gene that is suitable for use in the identification and selection of cells transformed with the cloning vector. Marker genes typically include genes that provide tetracycline resistance or ampicillin resistance. Suitable cloning vectors are described in J. Sambrook et al. (eds.). Molecular Cloning: A Laboratory Manual, 2nd Edition (Cold Spring Harbor Laboratory Press [1989]) and can be obtained, for example, from various sources.

The FcεR1α cDNA clone pGEM-3-110B-1, is described in A. Shimizu et al., Proc.Nat.Acad.Sci.USA 85 [1988] 1907-1911 and can be obtained from the American Type Tissue Collection (ATCC stock #67566). Single-stranded human liver cDNA can be obtained from Clontech (PCR-ready Quick Clone cDNA, Cat. D#7113-1). The sequence of HSA is available from GenBank under Accession #s: VOO495, JOOO78, LOO132, LOO133. HSA cDNA can be obtained by PCR amplification using oligonucleotides #24 and 25, as described in **Example 2**.

A polynucleotide encoding a suitable IgE-binding domain or HSA component DNA can be prepared using the polymerase chain reaction (PCR). PCR utilizes a single-stranded cDNA template and a mixture of oligonucleotide primers. The PCR procedure is perfomed via well-known methodology (see e.g. C.R.M. Bangham, "The Polymerase Chain Reaction: Getting Started" in Protocols in Human Molecular Genetics, Human Press [1991], Ch.I, p. 1-8). PCR kits and material for use in the kits are also commercially available from various sources. Kits and methods for using them are further described in e.g. USP 5'487'993).

DNA sequences encoding signal peptides can be added by PCR or if necessary using synthetic oligonucleotides that encode known signal peptide sequences. DNA sequences encoding a heterologous signal peptide are subcloned in frame with DNA sequences encoding the N-terminus of the fusion polypeptide.

Fusion of the polynucleotides may be accomplished by subcloning in intermediate vectors. Alternatively, one gene can be cloned directly into a vector containing the other gene. Linkers and adapters can be used for joining the DNA sequences.

Subcloning is performed in accordance with conventional techniques, such as by use of restriction enzyme digestion to provide appropriate termini, the use of alkaline phosphatase treatment to avoid undesirable joining of DNA molecules, and ligation with appropriate ligases. Techniques for such manipulation are described in the literature and are known in the art.

B Expression cloning of fusion polypeptides

The cloned fusion protein is then cleaved from the cloning vector and inserted into an expression vector. Suitable expression vectors typically contain

(1) prokaryotic DNA elements coding for a bacterial replication origin and an antibiotic resistance marker to provide for the growth and selection of the expression vector in a bacterial host;
(2) eukaryotic DNA elements that control initiation of transcription, such as a promoter; and
(3) DNA elements that control the processing of transcripts, such as a transcription termination/polyadenylation sequence.

Therefore, another aspect of the present invention concerns vectors, preferably plasmid vectors, for use in the expression of the fusion polypeptides of the invention, which contain the polynucleotide sequences described herein which code for the fusion polypeptides of the invention. Appropriate expression vectors which can transform procaryotic or eucaryotic cells include expression vectors comprising nucleotide sequences coding for the fusion molecules joined to transcriptional and translational regulatory sequences which are selected accoding to the host cells used. For expression in E. coli, vectors such as that described by M.W. Robertson, J. Biol. Chem. 268 [1993] 12736-12743 can be used. The product is expected to be disulfide-bonded, but not glycosylated. For expression in yeast, an expression vector such as pHIL-D2 supplied with the Invitrogen (San Diego, CA, USA) Pichia pastoris expression kit (catalogue # K1710-01) can be used. The protein product is expected to be disulfide-bonded and glycosylated. Other suitable yeast expression systems include Saccharomyces cerevisiae and Kluveromyces lactis. For expression in baculovirus, vectors such as pAC360, pVL1392 and pVL1393 (Invitrogen. San Diego, CA, USA) are useful for infection of insect cells, which would be expected to secrete a glycosylated and disulfide-bonded product.

The fusion polypeptide of the invention normally is a glycoprotein, particularly when expressed in mammalian cells, and the invention includes fusion polypeptides in any glycosylation, or disulfide bridging, state. In particular, mutational analysis suggests that N-linked glycosylation at the first, second and seventh positions of the N-linked glycosylation sites of the IgE receptor $\alpha$ chain (A. Shimizu et al., PNAS USA 85 [1988] 1907-1911, Figure 2) (corresponding to amino acid residues 46, 67 and 191 of SEQ.ID.NO.1), promotes biological activity of the IgE$^R$ molecule and monomers and dimers comprising it. The most preferred expression system is one in which any sugars added will be most similar to those in the native molecule from which the polypeptide is derived. Yeast and insect cells are known to modify glycoproteins differently from mammalian cells, whereas E. coli does not add sugar molecules after secretion. Therefore, while expression from any of these expression systems can yield a protein product which is useful for diagnostic applications (for example, the detection of an allergic condition), the most preferred form for expressing this product for use as a therapeutic molecule is expression in mammalian cells or possibly expression in the milk of transgenic mammals. For expression in a mammalian host, the transcriptional and translational regulatory signals used in an expression cassette may be derived from viral sources, such as adenovirus, bovine papilloma virus or simian virus, in which the regulatory signals are associated with a particular gene which has a high level of expression. Suitable transcriptional and translational regulatory sequences also can be obtained from mammalian genes, such as actin, collagen, myosin, and metallothionein genes. Transcriptional regulatory sequences include a promoter region sufficient to direct the initiation of RNA synthesis in a mammalian cell. Examples of typical mammalian cells are Chinese hamster ovary (CHO) cells, SV40-transformed monkey kidney cells (COS), HeLa. BHK, NIH Swiss mouse embryo cells, rat, monkey or human fibroblasts, or rat hepatoma cells.

For expression in mammalian cells, the preferred method is secretion from CHO cells. Neither CHO nor human cells add the $\alpha$1,3-linked galactose residues which are typical of expression in murine cells such as C127 and SP2/0 cells. Antibodies to this sugar linkage are present in human serum [C.F. Goochee et al., BioTechnol. 9 [1991] 1347-1355] and can affect the half-life, accessibility and clearance of recombinant products expressed from these murine cells. CHO, dhfr- cells are mutant for dihydrofolate reductase (DHFR), and therefore are unable to synthesize purines, thymidine and glycine de novo. The copy number of chromosomally integrated plasmids bearing wild type copies of the DHFR gene can be increased or amplified by exposing cells transformed with these plasmids to increasing levels of methotrexate, a folate analogue which competes for folate binding at the active site of the enzyme. A suitable vector for expression in CHO, dhfr-cells is pMT2 (R.J. Kaufman et al. EMBO J. 6 [1987] 187-193)]. The pMT2 vector has a wild type copy of the DHFR gene which is transcribed as a single mRNA with the foreign gene. Therefore, upon treatment of transformed CHO, dhfr- cells with increasing concentrations of methotrexate, both the foreign gene and the DHFR gene are co-amplified. The product secreted from these cells is expected to be disulfide-bonded and glycosylated in a mammalian pattern.

An expression vector can be introduced into host cells using a variety of techniques including calcium phosphate transfection, liposome-mediated transfection, electroporation, and the like. Preferably, transfected cells are selected and propagated wherein the expression vector is stably integrated in the host cell genome to produce stable transformants. The cells can be cultured, for example, in DMEM media. The polypeptide secreted into the media can be recovered by standard biochemical approaches following transient expression 24 - 72 hours after transfection of the cells or after establishment of stable cell lines following selection by e.g. antibiotic resistance.

Conventional methods for recovering an expressed polypeptide from a culture include fractionation of the polypeptide-containing portion of the culture using well known biochemical techniques. For instance, the methods of gel filtration, gel chromatography, ultrafiltration, electrophoresis, ion exchange or affinity chromatography, such as are known for protein fractionations, can be used to isolate the expressed proteins found in the culture. In addition, conventional immunochemical methods, such as immunoaffinity or immunoabsorption can be performed. Techniques for transformation, culture, amplification, screening and product production and purification are also well-known (see e.g. J. Sambrook et al. [1989], supra; R.J. Kaufman, Genetic Engineering: Principles and Methods, Plenum Press, Vol. 9 [1987] 156-198).

The fusion polypeptides of the invention are indicated for use therapeutically to treat mammalian, and in particular, human, patients suffering from allergies. The IgE-binding domain of the fusion polypeptides competes for IgE with the IgE receptor naturally present on mast cells, basophils and Langerhans cells, so that IgE is bound to the administered protein and unable to bind to these allergy effector cells to mediate the allergic response. The IgE-binding domain also competes with the IgE receptor, FcεRI, by binding to auto-antibodies to FcεRI.

Therefore the present invention provides a pharmaceutical composition for competitively binding IgE (and/or autoantibodies to FcεRI), and/or inhibiting production of IgE. and thus for suppressive and/or preventative treatment of IgE- or IgE-receptor-mediated disorders, and more specifically, allergy and conditions associated with allergy, such as atopic dermatitis, atopic asthma and chronic urticaria.

By "IgE- or IgE receptor- mediated disorders" is meant disorders associated with the binding of the cell-bound IgE receptor, FcεRI, to IgE or to auto-antibodies to FcεRI, e.g. type allergic reactions ("hyper-IgE syndrome") such as bronchial asthma, atopic asthma, hay fever, pollen allergy, allergic rhinitis, atopic dermatitis, eczema, anaphylaxis, as well as chronic urticaria, and also non-allergic Kimura's disease, and other pulmonary, dermatological or autoimmune diseases.

In particular, atopic dermatitis, one of the most dramatic manifestations of atopy, is a chronic inflammatory skin disease associated with high serum IgE levels and a sensitization to various environmental allergens (M.-A. Morren et al., J. Am. Acad. Dermatol. 31 [1994] 467-473). Current treatment of atopic dermatitis concentrates on the use of steroid-containing creams, and severe cases of atopic dermatitis have been successfully treated with cyclosporin A (H. Granlund et al., Br. J. Dermatol. 132 [1995] 106-112), but the side effects restrict this treatment to a minority of the patients. An agent that inhibits the functions of IgE such as mast cell degranulation and IgE-mediated antigen presentation by B cells and other antigen presenting cells would be superior to any presently known treatment of atopic dermatitis and in addition also be useful for other milder forms of allergy.

In addition to atopic dermatitis and atopic asthma, the polypeptides of the invention can be used to treat or prevent chronic urticaria (CU), in which mast cell degranulation through activation of FcεRIα plays a role. The fusion polypeptides of the invention can clear circulating autoantibodies against FcεRIα, in contrast to anti-IgE monoclonal antibodies alternatively proposed for treatment of the disease.

The polypeptides may be administered in the form of a pharmaceutical composition comprising a polypeptide of the invention, preferably an unmodified polypeptide, or a pharmaceutically acceptable salt thereof, in a pharmaceutically acceptable carrier or diluent.

The term "salt" refers in particular to pharmaceutically acceptable salts prepared from pharmaceutically acceptable, non-toxic acids to form acid addition salts of e.g. an amino group of the polypeptide chain, or from pharmaceutically acceptable non-toxic bases to form basic salts of e.g. a carboxyl group of the polypeptide chain. Such salts may be formed as internal salts and/or as salts of the amino or carboxylic acid terminus of the polypeptide of the invention.

Suitable pharmaceutically acceptable acid addition salts are those of pharmaceutically acceptable, non-toxic organic acids, polymeric acids, or inorganic acids. Examples of suitable organic acids comprise acetic, ascorbic, benzoic, benzenesulfonic, citric, ethanesulfonic, fumaric, gluconic, glutamic, hydrobromic, hydrochloric, isothionic, lactic, maleic, malic, mandelic, methanesulfonic, mucic, nitric, oxalic, pamoic, pantothenic, phosphoric, salicylic, succinic, sulfuric, tartaric and p-toluenesulfonic, as well as polymeric acids such as tannic acid or carboxymethyl cellulose. Suitable inorganic acids include mineral acids such as hydrochloric, hydrobromic, sulfuric, phosphoric and nitric acid.

Examples of suitable inorganic bases for forming salts of a carboxyl group include the alkali metal salts such as sodium, potassium and lithium salts; the alkaline earth salts such as calcium, barium and magnesium salts; and ammonium, copper, ferrous, ferric, zinc, manganous, aluminum and manganic salts. Preferred are the ammonium, calcium, magnesium, potassium, and sodium salts. Examples of pharmaceutically acceptable organic bases suitable for forming salts of a carboxyl group include organic amines, such as trimethylamine, triethylamine, tri(n-propyl)amine, dicyclohexylamine, β-(dimethylamino)ethanol, tris(hydroxymethyl)aminomethane, triethanolamine, β-(diethylamino)ethanol, arginine, lysine, histidien, N-ethylpiperidine, hydrabamine, choline, betaine, ethylenediamine, glucosamine, methylglucamine, theobromine, purines, piperazines, piperidines, caffeine and procaine.

Acid addition salts of the polypeptides may be prepared in conventional manner by contacting the polypeptide with one or more equivalents of the desired inorganic or organic acid such as hydrochloric acid. Salts of carboxyl groups of the peptide may be conventionally prepared by contacting the peptide with one or more equivalents of a desired base such as a metallic hydroxide base, e.g. sodium hydroxide; a metal carbonate or bicarbonate base such as sodium carbonate or sodium bicarbonate; or an amine base such as triethylamine or triethanolamine.

The invention thus also concerns pharmaceutical compositions comprising a novel fusion polypeptide as defined above or a pharmaceutically acceptable salt thereof, together with a pharmaceutically acceptable carrier or diluent. The carrier is preferably a sterile, pyrogen-free, parenterally acceptable liquid. Water, physiological saline, aqueous dextrose, and glycols are preferred liquid carriers or diluents, particularly (when isotonic) for injectable solutions. The composition may be a conventionally prepared lyophilizate.

The compositions can be administered systemically, i.e. parenterally (e.g intramuscularly, intravenously, subcutaneously or intradermally), or by intraperitoneal administration. For parenteral administration, it is preferred that the fusion polypeptides be essentially soluble in patient serum or plasma, e.g. that at least 1 milligram of polypeptide is soluble in one mililiter of serum or plasma.

The compositions can also be administered by known techniques for topical administration. Examples of suitable dosage forms include sprays, opthalmic solutions, nasal solutions and ointments. For example, a spray can be manufactured by dissolving the peptide in an appropriate solvent and putting it in a spray to serve as an aerosol for commonly employed inhalation therapy. An opthalmic or nasal solution can be manufactured by dissolving the active ingredient in distilled water, adding any auxiliary agent required, such as a buffer, isotonizing agent, thickener, preservative, stabilizer, surfactant or antiseptic, adjusting the mixture to pH 4 to 9. Ointments can be obtained e.g. by preparing a composition from a polymer solution, such as 2 % aqueous carboxyvinyl polymer, and a base, such as 2 % sodium hydroxide, mixing to obtain a gel, and mixing with the gel an amount of purified fusion polypeptide.

The composition is preferably administered subcutaneously or intravenously, and most typically, subcutaneously.

The invention also comprises a method of treatment of allergic conditions comprising administration of a therapeutically effective amount of a fusion polypeptide of the invention or a pharmaceutically acceptable salt thereof to a patient in need of such treatment. The method of treatment is practiced during the course of an allergic disease state (i.e. when the relief of symptoms is specifically required); or as a continuous or prophylactic treatment (i.e. prior to the onset of an anticipated IgE- or IgE-receptor - mediated disorder, such as an allergic reaction).

The effective dosage in accordance herewith can vary over a wide range taking into consideration e.g. the degree or severity of the condition being treated, the age, sex and condition of the subject, the length of treatment, and the potency of the particular fusion protein, factors which are determinable by conventional methods.

Since individual subjects widely vary in their IgE (as well as antibody to FcεRI) content, a therapeutically effective dosage in accordance herewith can best be described as being between $5 \times 10^2$ and $1 \times 10^4$ times the total content of serum IgE and antibodies to FcεRI, on a molar scale. The patient may be treated on a daily basis in single or multiple administration. The composition may also be administered on a per month basis (or at such weekly intervals as may be appropriate), also in either single or multiple administrations.

For an average subject (70 kg), a suitable monthly dosage can range from a lower dosage of about 0.5 mg per month to an upper dosage of about 500 mg per month, preferably of from about l mg to about 300 mg, more preferably of from about 20 mg to about 250 mg, per month, of the fusion polypeptide of the invention, such as the dimer of Example 7, conveniently administered in divided dosages once or twice a month. Higher dosage ranges, e.g. 500 mg per month to 2 g per month, may be indicated in patients having high serum IgE concentrations or in early treatment phases.

The dosage and timing of administration may vary. Initial administrations of the composition may be at higher dosages within the above ranges, and administered more frequently than administrations later in the treatment of the disease. Appropriate dosages may be determined by measuring the content of IgE and antibodies to FcεRI in the patient's serum, as indicated above. For example, early in the course of disease, the fusion polypeptide of the invention, such as the dimer of Example 7, may be administered in weekly doses of 200-500 mg of polypeptide in the average patient (70 kg). After clearance of serum IgE and antibody to FcεRI, the treatment regimen may be reduced to weekly treatments or treatments every other week, with dosages ranging from 50 μg to 100 mg of polypeptide per treatment.

The compositions of the present invention can be administered either alone or in combination with other compounds of the present invention or further pharmaceutical agents such as antihistamines or corticosteroids.

The invention also comprises the use of the fusion polypeptides of the invention in an in vitro diagnostic assay of any standard format, e.g. ELISA, to determine the level of IgE or auto-antibodies to FcεRI (e.g. in chronic urticaria patients) in a biological sample obtained from a human patient, e.g. blood or tissue samples. The HSA component advantageously facilitates binding and detection of IgE or autoantibodies to FcεRI in an ELISA formatted assay. The amount of IgE or auto-antibodies present in the sample can serve as a measure of the allergic response of the patient to a substance to which the patient has been exposed. IgE or auto-antibody levels can also be measured to determine the efficiency of anti-allergy therapies, and to monitor a patient's allergic status over time.

The invention further comprises a method of performing gene therapy in humans using a polynucleotide encoding a fusion polypeptide of the invention, for treatment of IgE- or IgE receptor- mediated disorders. The gene therapy method comprises modifying cells of a patient by introducing therein a polynucleotide encoding a fusion polypeptide of the invention and expressing the polypeptide from such cells. For example, somatic cells may first be removed from a patient, then genetically modified in culture by insertion of the polynucleotide, and the resultant modified cells reintroduced into the patient, whereby a polypeptide of the invention is expressed by the cells of the patient.

Alternatively, the cells may be modified in vivo by direct insertion of vector DNA encoding the polypeptide.

Suitable cells for modification include endothelial cells or leukocytes. For gene therapy applications the polynucleotide of the invention is preferably under the control of a regulable (e.g. inducible) promoter. Expression of the polypeptide may thereby be made dependent on exposure of the patient to an exogenous factor using known regulable promoter systems.

The invention also includes utilizing the methods of gene therapy to prepare non-human somatic recombinant or non-human transgenic animals expressing the fusion polypeptides of the invention, e.g. into milk. Such modified non-human animals also form part of the invention. Examples of useful non-human animals include mice, rats, rabbits, pigs, sheep, goats and cattle, all of which have been made transgenic using standard techniques (e.g., D.R. Hurwitz et al., Transgenic Research 3 [1994] 365-375). The non-human animals may be used for modelling purposes or actual production of a protein. In particular, the invention concerns a transgenic mouse, goat, cow or pig expressing a fusion polypeptide of the invention. Methods for making such non-human animals are well known. A polynucleotide encoding the fusion protein of the invention can be introduced into the somatic cells of the non-human animals, in vitro or in vivo, to produce somatic recombinant non-human animals which are modified genetically but which cannot pass the genetic modification on to offspring Alternatively, the polynucleotide can be inserted into cells of non-human embryos for production of non-human transgenic animals able to pass on the capability of expressing the proteins of the invention to offspring.

Transfection of non-human cells for gene therapy can be accomplished in conventional manner, e.g. by electroporation, calcium phosphate precipitation, a lipofectin-based procedure, intramuscular injection, microinjection or through use of a "gene gun." Plasmid-based vectors preferably contain a marker such as the neomycin gene for selection of stable transfectants with the cytotoxic aminoglycoside G418 in eukaryotic cells.

Infection is accomplished by incorporating the genetic sequence for the fusion polypeptide into a retroviral vector. Various procedures are known in the art for such incorporation. One such procedure which has been widely used employs a defective murine retrovirus for packaging the retrovirus, and an amphotropic packaging cell line to prepare infectious amphotropic virus for use in infecting the non-human target donor cells.

Further characterization may be effected e.g. as follows:

### In vivo determination of serum half life

General procedure:

Female SKHI/hr/hr Charles River mice weighing approximately 25 g are injected intravenously with test proteins diluted in sterile $Ca^{+2}$, $Mg^{2+}$ - free PBS. The mice are divided into groups as follows:

- Group (i) receiving 130 $\mu g$ (1 nmole) of fusion polypeptide, e.g. the dimer $IgE^R$ - $L_1$ - HSA II - $L_2$ - $IgE^R$ prepared as in Example 7, or
- Group (ii) receiving 60 $\mu g$ $IgE^R$ (2 nmoles), or
- Group (iii) receiving 65 $\mu g$ HSA I (1 nmole).

100 $\mu l$ of blood are taken from each mouse at 10 minutes, 30 minutes, 3 hours, 6 hours and 12 hours after injection. Serum is prepared by centrifugation. Serum concentration levels of the various proteins are determined by a) IgE receptor ELISA binding assay; b) inhibition ELISA; or c) HSA Sandwich ELISA, as follows:

a) $IgE^R$ ELISA binding assay:

IgE serum concentration is determined by detection of IgE binding by the following Sandwich ELISA: 200 ng human IgE is immobilized in COSTAR Strip Plate-8 wells (Cambridge. MA, USA) in 100 $\mu l$ coating buffer in a humidifed chamber at 4°C overnight. Each well is washed twice with 300 $\mu l$ $Ca^{2+}$, $Mg^{2+}$ - free PBS, pH 7.2. The plates are blocked for one hour at room temperature with 200 $\mu l$ $Ca^{2+}$, $Mg^{2+}$ - free PBS containing 5% BSA (Sigma). After washing twice

with 300 µl Ca$^{2+}$, Mg$^{2+}$ - free PBS containing 0.05 % Tween 20 (PBST), samples diluted in 100 µl of 1:10 diluted (in Ca$^{2+}$, Mg$^{2+}$- free PBS) mouse serum are added and incubated for one hour at room temperature.

The wells are washed twice with 300 µl PBS and incubated with 100 µl (Ing) of a monoclonal anti-human IgE receptor antibody such as 5H5/F8 for one hour at room temperature. Again, the wells are washed twice with 300 µl PBST and incubated with 100 µl of goat anti-mouse IgG-HRP (Biorad, 1:2000 diluted in Ca$^{2+}$, Mg$^{2+}$- free PBS) for 1 hour at room temperature. Plates were washed three times with 300 µl of PBST, and horse radish peroxidase (HRP) conjugates are detected with 100 µl of ABTS (Biorad) substrate. The reaction is stopped after 5 minutes with 100 µl of 3 % oxalic acid. Color intensities are measured with an EASY READER photometer at 405 nm.

b) Inhibition ELISA:

100 ng FcεRIα is immobilized in Nunc 96 well Immunoplates (F96 cert. Maxisorb) in 100 µl coating buffer (0.1 M NaHCO$_3$, 0.01 % NaN$_3$ pH:9.6) in a humidified chamber at 4˚C overnight. Each well is washed 4 times with 300 µl PBS, 0.05 % Tween 20 (washing buffer). To different sets of wells, either a negative control (50 µl of mouse serum diluted 1:25 in PBS, 0.05 % Tween 20. 2 % FCS), a dilution series of the fusion polypeptide standard, e.g. IgE$^R$- L$_1$- HSAII - L$_2$- IgE$^R$ standard (dilutions from 400 ng/ml to 1.6 ng/ml) or dilution series of the samples are added. The standard and the samples are diluted in mouse serum diluted 1:25 in PBS, 0.05 % Tween 20, 2 % FCS. Immediately afterwards 50 µl of 400 µg/µl of human IgE-Biotin conjugate in dilution buffer is added and mixed. The final mouse serum dilution in the incubation mixture is 1:50.

After incubation for two hours at 37˚C, the plates are washed 4 times with 300 µl washing buffer, and 50 µl steptavidine-alkaline phosphatase conjugate (Gibco) diluted 1:1000 in dilution buffer is added and incubated for one hour at 37˚C. The plates are washed 4 times with 300 µl washing buffer and after addition of 100 µl substrate (1 mg/ml p-nitrophenyl-phosphate in diethanolamine buffer, pH 9.8 [BIORAD]) the reaction is stopped after incubation for 30 minutes at 37˚C with 50 µl of 2 M NaOH. Optical densities are measured with a BIOMEK-1000 workstation photometer at 405 nm. The quantitative evaluation from the standard curves (4-parameter logistic curve fitting) is made with the Beckman IMMUNOFIT ELISA evaluation program. Calculation:

$$\text{\% binding} = [\text{OD (sample or standard value)} / \text{OD (buffer value)}] \times 100$$

c) HSA Sandwich ELISA:

500 ng monoclonal anti-mouse HSA (HSA-9) is immobilized in Nunc 96 well Immunoplates (F96 cert. Maxisorb) in 100 µl coating buffer (0.1 M NaHCO$_3$, 0.01 % NaN$_3$ pH:9.6) in a humidified chamber at 4˚C overnight. Each well is washed 4 times with 300 µl washing buffer (PBS, 0.05 % Tween 20). 100 µl of 1:100 diluted mouse serum (PBS, 0.05 % Tween 20, 2 % FCS = dilution buffer) as negative control, or 100 µl standard (1µg/ml - 2 ng/ml human serum albumin, KABI) or 100 µl of sample diluted in 1:100 diluted mouse serum is added. After incubation for two hours at 37˚C, the plates are washed 4 times with 300 µl washing buffer, and 100 µl of 1 ng/µl rabbit anti-HSA-Biotin conjugate diluted in dilution buffer is added. The anti-HSA-Biotin conjugate is purified by immunoaffinity chromatography with CH-Seph4B-HSA, and cross reactivities with mouse serum are removed by immunosorbtion with mouse serum-agarose. After incubation for two hours at 37˚C, the plates are washed 4 times with 300 µl washing buffer, and 50 µl streptavidin-alkaline phosphatase conjugate (Gibco) diluted 1:1000 in dilution buffer is added and incubated for one hour at 37˚C. The plates are washed 4 times with 300 µl washing buffer and after addition of 100 µl substrate (1mg/ml p-nitrophenylphosphate in diethanolamine buffer, pH 9.8, BIO-RAD), the reaction is stopped after incubation for 15 minutes at 37˚C with 50 µl 2M NaOH. Optical densities are measured with a BIOMEK-1000 workstation photometer at 405 nm. The quantitative evaluation from the standard curve (4-parameter logistic curve fitting) is made with the Beckman IMMUNOFIT ELISA evaluation program.

Results:

Concentrations for the dimeric fusion polypeptide, IgE$^R$ - L$_1$ - HSA II - L$_2$ - IgE$^R$, the free IgE$^R$ (referred to as "free alpha chain") or HSA I (referred to as "HSA") as a function of elapsed time after injection, are given in pmoles/ml serum in **Figure 1(A)** and (**B**).

Figure 1(B) shows that the free receptor is only detectable in mouse serum for about 10 minutes, whereas the dimeric fusion polypeptide is still detectable about 12 hours after administration.

**Figure 1(C)** shows the relative clearance kinetics for HSA and the dimeric fusion Polypeptide. After stabilization of tissue-blood distribution in the first 10 minutes, the dimer and HSA show an almost identical clearance curve. This confirms that the serum half life of an IgE-binding domain can be substantially prolonged by fusion to HSA.

**Inhibition of passive cutaneous anaphylaxis (PCA) in mice**

General procedure:

(a) Administration of polypeptide:

Serial dilutions of 10, 50 or 500 $\mu$g/kg of fusion polypeptide, e.g. $IgE^R$ - $L_1$ - HSA II - $L_2$ - $IgE^R$ obtained from CHO cells in Example 7, are intravenously injected into female SKH1/hr/hr Charles River mice weighing approximately 25 g at varying intervals prior to sensitization.
Three groups of mice are established depending on the amount of polypeptide injected prior to sensitization (i.e. 10 $\mu$g/kg, 50 $\mu$g/kg or 500 $\mu$g/kg). A fourth group of control mice receive 200 $\mu$g/kg PBS intravenously instead of polypeptide. The four groups of mice are each divided into three sub-groups, which differ by the interval between intravenous injection of the compound and intracutaneous sensitization with IgE [step (b) below]. The tested intervals are: 5 minutes, 15 minutes and 30 minutes.

(b) Intracutaneous sensitization:

The mice are anaesthetized and sensitized into the back skin by 4 intradermal injections of 5 ng each monoclonal mouse anti-dinitrophenyl (DNP) IgE antibody in 10ml PBS (BioMakor, Rehovot, Israel). In the control group, saline is injected intradermally in one site.

(c) Allergen challenge:

90 minutes after sensitization, the mice are anesthesized again and challenged by intravenous injection of a solution containing 50 $\mu$g of dinitrophenyl-bovine serum albumin (DNP-BSA) (Calbiochem-Behring, San Diego, USA) containing 1% Evans blue. The PCA response was quantified by measuring the diameter of the stained test site due to extravasation.

Results:

These are depicted in the bar graph in **Figure 2** for the mature fusion polypeptide of Example 7 (amino acids $Val_{26}$-$Leu_{978}$ of SEQ.ID.NO.3). At a concentration of 500 $\mu$g/kg, the dimeric fusion polypeptide completely blocks PCA at all time points of application. At 50 $\mu$g/kg, treatment at 30 minutes prior to challenge gives a statistically significant reduction of 36 %. At 15 minutes prior to challenge, a trend is seen with administration of both 10 and 50 $\mu$g/kg of dimer. Thus, the dimeric fusion polypeptide is efficacious in preventing PCA.
The procedures and techniques for carrying out the present invention are known in the art. Insofar as their preparation is not particularly described herein, the compounds, reagents, vectors, cell-lines, etc. to be used are known and readily available or may be obtained in conventional manner from known and readily available materials, or equivalent materials may be prepared in conventional manner from known and readily available materials.
The following non-limitative **Examples** illustrate the invention. All temperatures are in degrees Centigrade.

**Materials and methods**

PCR amplification:

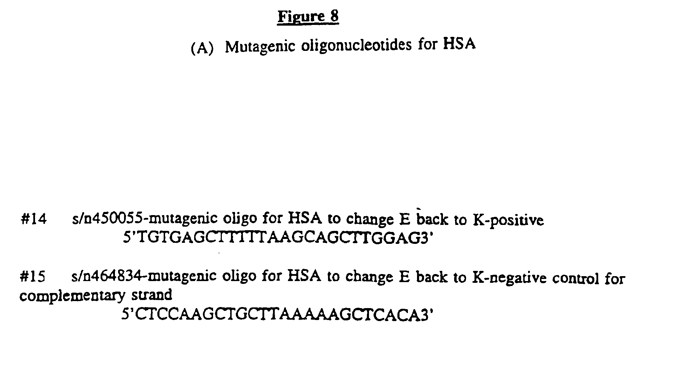

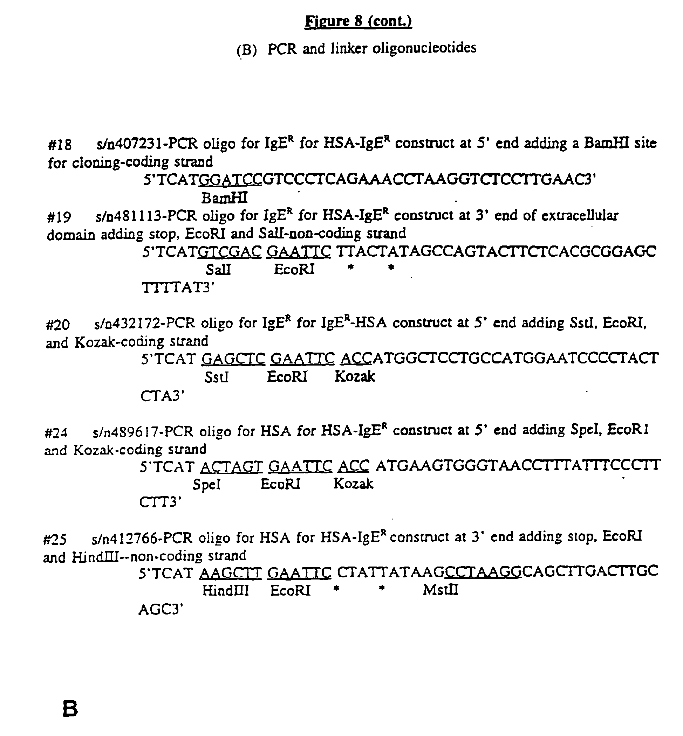

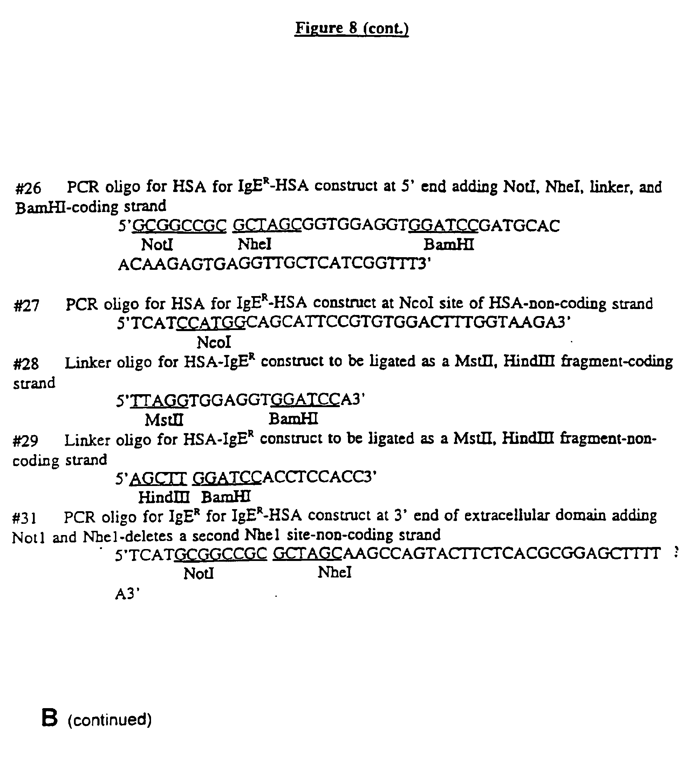

De novo chemical synthesis of the primers of the invention can be conducted using any suitable method, such as, for example, the phosphotriester or phosphodiester methods.
Methods and systems for amplifying a specific nucleic acid sequence are described in USP 4'683'195 and USP 4'683'202; and in Polymerase Chain Reaction, H.A. Erlich et al., Eds., Cold Spring Harbor Laboratory Press [1989].
Following PCR, the DNA fragments are excised and purified using the QiaEx protocol (Qiagen, Inc., Chatsworth, CA, USA), then subcloned into a TA vector [TA Cloning® Kit (Invitrogen) (product literature, Version 2.2)]. The primers used in generating PCR amplified nucleic acids are set forth in **FIG. 8.** DNA is sequenced using the Sequenase method (USB, Cleveland, OH, USA).

Plasmids and Reagents:

The FcεR1α cDNA clone, pGEM-3-110B-1 (A. Shimizu et al., Proc.Nat.Acad.Sci.USA· 85 [1988] 1907-1911) is obtained from the American Type Tissue Collection (ATCC stock #67566). Single-stranded human liver cDNA is obtained from Clontech (PCR-ready Quick Clone cDNA, Cat. D#7113-1). The sequence of HSA is available under GenBank Accession #s VOO495, JOOO78, LOO132 and LOO133. Restriction enzymes are obtained from Boehringer-Mannheim or Gibco/BRL. Taq DNA polymerase is obtained from Perkin-Elmer Cetus (PECI) or from Boehringer-Mannheim. The SK vector is obtained from Stratagene.

pHIL-D2 is available from Invitrogen (San Diego, CA, USA; Catalog no. K1710-01 [1994]) (see "Pichia Expression Kit - Protein Expression - A Manual of Methods for Expression of Recombinant Proteins in Pichia pastoris - Version 3.0" [December 1994]) (hereinafter referred to as the "Invitrogen Manual").

The pXMT3 vector is derived from pMT2 (Sambrook et al. (Eds.) [1989] supra) by cloning the PstI to EcoRI linker from pUC8 (Pharmacia) into the PstI and EcoRI sites of pMT2 (R.J. Kaufman et al. [1987] supra).

Standard techniques as used below are described in Sambrook et al. (Eds.) [1989] supra.

**Example A: TA Cloning Vector**

Plasmid pCR2 is ligated separately with each of the PCR amplification products obtained in Examples 1 and 2. The ligation reactions are performed using T4 DNA ligase in the following reaction mixture:

    25 mM Tris-HCl (pH 7.8)
    10 mM MgCl$_2$
    I mM DTT
    1 mM ATP
    50 ng vector DNA
    100-200 ng PCR reaction products (unpurified)
    4 units T4 DNA ligase (New England Biolabs)

Each reaction mixture is maintained at 15˚ for 18 hours before being transformed into competent cells.

**Example 1: PCR amplification and cloning of FcεRIα cDNA**

FcεR1α cDNA is purified from pGEM-3-110B-1 using the Qiagen method. Then:

(A) For preparing an HSA-leading construct, PCR amplification of FcεR1α cDNA is carried out with oligonucleotides #18 and #19 **(FIGS. 4, 8)** using the following reaction mixture:

    I μl (50 ng) of FcεRIα cDNA;
    50 pmoles of each of oligonucleotides #18 and #19
    5 μl 10x PCR buffer (PECI)
    0.5 μl 20 mM dNTP stock solution = 200 μM final dNTPs concentration
    0.5 μl (2.5 U) Taq DNA polymerase (PECI)
    water to 50 μl.

The reaction mixture is overlaid with mineral oil to prevent evaporation, and thermocycled in a Perkin Elmer Cetus DNA thermocycler model 480. Cycling conditions for this reaction are: heat to 95˚ for 5 minutes, then 30 cycles of 94˚ for 1.5 minutes, 53˚ for 2 minutes, 72˚ for 3 minutes, followed by a three minute extension at 72˚ and an overnight soak at 4˚.

Following electrophoresis an amplified product of -550 bp is confirmed by ethidium bromide staining. The fragment is subcloned into PCR2 vector to yield pEK1 encoding IgE[R] (**Figs. 4, 8B**).

(B) For preparing an IgE-leading construct, PCR amplification of the FcεRIα cDNA is carried out according to the procedure of (A) above except that oligonucleotides #20 and #31 (**FIG. 8B**) are used. Following electrophoresis on 0.7 % agarose gel, an amplified product of ~600 bp is confirmed by ethidium bromide staining. The fragment is subcloned into PCR2 vector to form IgER/TA#1 encoding pre-IgE[R] (**FIG. 4**) .

(C) For preparing a construct encoding pre-IgE[R] in order to express the mature, truncated protein for use as a control in assays, PCR amplification of FcεRIα cDNA is carried out as in (A) above with oligonucleotides #20 and #19 **(FIG. 8B).** Following electrophoresis, an amplified product of ~620 bp is confirmed by ethidium bromide staining. The PCR fragment is excised and subcloned into PCR II vector to provide IgERFL/TA #34, encoding pre-IgE[R]

followed by a stop codon (**FIG. 4**).

## Example 2: PCR amplification and cloning of human serum albumin cDNA

For obtaining a sequence encoding prepro-HSA II, PCR amplification of full length human serum albumin cDNA is carried out with oligonucleotides #24 and #25 **(FIG. 8B),** following the general procedure of Example 1(A). The resulting clone HSA/TA#1 had the sequence most closely resembling the sequence in Genbank. In this clone, seven mutations in the wobble position and one mutation which resulted in a change from lysine to glutamic acid at base 1333 were detected. The seven mutations in the wobble position were: base 309: A to T; base 744: A to G; base 795: G to A; base 951: G to A; base 1320: C to T, base 1569: A to C; base 1584: G to A (with reference to HSA/TA#1). The lysine to glutamic acid mutation was corrected back to the sequence in Genbank using site-directed mutagenesis with the oligonucleotides shown in **FIG. 8A** and the Bio-Rad Mutagene Phagemid in vitro mutagenesis kit (Biorad, Cat #170-3581). This method relies on the incorporation of uracil residues in the parental strand and their subsequent removal in the mutagenized strand (T.A. Kunkel, Proc.Nat.Acad.Sci.USA 82 [1985] 488-492). Because the point mutations in the wobble position do not alter the native amino acid sequence of the encoded protein, the above seven mutations were not corrected. Following electrophoresis, an amplified product of -1.8 kb is confirmed by ethidium bromide staining.

The 1.8 kb product is subcloned into pCR2 vector to form HSA/TA mut #16, which is verified by DNA sequencing to contain the complete prepro-HSA II sequence. HSA/TA mut #16 is subcloned into Bluescript SK as a SpeI, HindIII fragment, yielding HSA/SK #17 **(FIG. 5).**

(A) For preparing an <u>HSA-leading construct</u>, HSA/SK#17 is digested with MstII and HindIII to remove the nucleotide sequence encoding the 3'-terminal amino acid (Leu$_{609}$); and an oligonucleotide encoding linker $L_2$, as defined above, is introduced at the 3'-terminus of the linearized HSA/SK#17 by kinasing oligonucleotides #28 and #29 (FIG. 8B) and annealing and ligating these fragments into the MstII/HindIII sites of the linearized HSA/SK#17, to yield pEK7 encoding prepro-HSA II fused to $L_2$ **(FIG. 8B).** Miniprep DNA is checked by BamHI digestion and by sequencing.

(B) For preparing an <u>IgE-leading construct,</u> HSA/SK#17 encoding prepro-HSA II is amplified by PCR with oligonucleotides #26 and #27 (**FIGS. 5, 8**). Oligonucleotide #26 removes the prepro sequence from HSA, and adds an oligonucleotide encoding $L_1$ at the 5' end of HSA. Oligonucleotide #27 ends at a naturally occurring, unique NcoI site at about nucleotide position 800 of the HSA coding sequence. PCR amplification is carried out according to the procedure in Example 1 (A). An approximately 800 bp fragment isolated by gel electrophoresis and Qia is subcloned into a pCR2 vector, to provide clone HSA Nco/TA #13, the sequence of which is verified by DNA sequencing. The NcoI to NotI fragment from this clone is subcloned into cut NcoI and NotI HSA/SK #17 DNA, yielding HSA/SK #5 **(FIG. 5)** encoding $L_1$ linked to the 5'-terminus of cDNA encoding HSA II.

(C) For expressing <u>HSA alone,</u> the HSA/SK #17 construct prepared above is employed.

## Example 3: Fusion construct HSA-IgER/SK#22 encoding prepro-HSA + Linker + IgE<sup>R</sup>

pEK7 (containing prepro-HSA II cDNA + oligonucleotide for $L_2$) and pEK1 (containing IgE$^R$) are digested with BamHI and SalI. The obtained 1.8 kb fragment from pEK7 is phosphatased and then ligated to the 550 Kb fragment obtained from pEK1. One positive miniprep, #23, was prepared but was contaminated with another unknown plasmid which prevented recovery of the HSA-IgE$^R$ band. Therefore, the 2.4 kb SpeI to SalI fragment containing the HSA - IgE$^R$ fusion and the 2.9 kb fragment containing the vector DNA were purified from miniprep#23 and ligated together, resulting in clone HSA - IgE/SK#49 **(FIG. 9)** [vector sites were found to be missing from either end of the subcloned region, and accordingly, the 2.4 kb SpeI to SalI fragment from HSA-IgE/SK#49 was subcloned into SpeI plus SalI - cut Bluescript SK, resulting in HSA-IgE/SK#22 (not shown in the Figures)].

The sequence of the junction of the HSA and linker with the IgE receptor DNA and the sequence of the ends of the fusion with the vector DNA were as expected in HSA-IgE/SK#22 as verified by DNA sequence analysis.

## Example 4: Fusion construct IgE·HSA/SK#1 encoding IgE<sup>R</sup> + prepro-HSA II

HSA/SK #5 and IgE$^R$/TA #1 are digested with SstI and NheI. The 600 bp fragment from IgE/TA #1 is purified by gel electrophoresis and ligated into the cut and phosphatase-treated HSA/SK #5, yielding clone IgE-HSNSK#1 **(FIG. 10).**

## Example 5: Fusion construct R-H-R/SK #50 encoding pre - IgE<sup>R</sup> - L<sub>1</sub>-HSA II -L<sub>2</sub>-IgE<sup>R</sup>

The PstI site unique to the HSA region of IgE$^R$-HSA/SK#1 and HSA-IgE$^R$/SK#49 is used to join IgE$^R$ and prepro - HSA II via the oligonucleotide for $L_2$. HSA/IgE$^R$/SK #49 and Bluescript SK are digested with PstI and SalI. A 1.2 kb fragment containing the 3' portion of HSA II, the linker and the IgE$^R$ sequence are ligated into PstI plus SalI- cut Bluescript DNA, resulting in HSA-IgE$^R$ Pst Sal/SK #37 **(FIG. 11),** which is digested with PstI and KpnI, and the 1.2 kb fragment is prepared.

IgE$^R$-HSA/SK#1 DNA is digested with PstI and KpnI, and a 4.8 kb fragment containing the vector, IgE$^R$, linker and 5' half of HSA are isolated, phophatase-treated, and ligated to the 1.2 kb fragment from HSA-IgE$^R$ Pst Sal/SK #37. The resultant dimeric construct R-H-R/SK #50 is obtained **(FIG. 11).**

## Example 6: HSA II - L$_2$. IgE$^R$ monomeric fusion polypeptides by transfection and culture of Pichia pastoris

Plasmid MB#2 encoding HSA II - L$_2$ - IgE$^R$ is prepared by cutting the plasmid HSA/SK#49 with EcoRI and isolating the 2.4 kb fragment encoding the fusion protein.

This fragment is ligated into the unique EcoRI site of the Pichia pastoris expression vector pHIL-D2 (Invitrogen), after digestion of plasmid pHIL-D2 with EcoRI and alkaline phosphatase treatment. The resultant MB#2 plasmid is linearized by digestion with NotI and transformed into his4 GS 115 cells as described in the "Invitrogen Manual". His+ transformants are screened for growth on methanol. Strains exhibiting slow growth on methanol are grown in minimal glycerol medium as specified in the "Invitrogen Manual" to stationary phase, transferred by centrifugation to buffered complex methanol medium and grown for 4 days. The supernatant from the cells is then assayed by ELISA for the presence of HSA and for IgE-binding ability. The IgE-binding ability of the product obtained secreted from P. pastoris was equivalent on a molar basis to the HSA concentration and fully biologically active.

## Example 7: IgE$^R$- L$_1$ - HSA II - L$_2$ - IgE$^R$ dimeric fusion polypeptide by transfection and culture of CHO cells

Plasmid pXMT3-RIα-HSA-RIα (containing the polynucleotide of **SEQ. ID. NO. 4** encoding pre- IgE$^R$- L$_1$- HSA II - L$_2$ -IgE$^R$) is prepared by digestion of R-H-R/SK #50 (see Example 5) with EcoRI and isolation of the 3 kb EcoRI fragment encoding the dimeric fusion polypeptide. This fragment is ligated into the unique EcoRI site of pXMT3 after digestion of pXMT3 with EcoRI and alkaline phosphatase-treatment.

The plasmid is transfected into CHO DUKX B 11 cells. These cells lack a functional dhfr-gene (dihydrofolate reductase) required for nucleoside synthesis. Therefore, cells are maintained in Alpha+ medium (MEM ALPHA MEDIUM with ribonucleosides and deoxyribonucleosides/Gibco) containing 10 % fetal calf serum (FCS). For transfection, the cells are washed twice in Ca$^{++}$-Mg$^{++}$-free PBS (CMF-PBS) and cell concentration is adjusted to 2x10$^6$ cells/ml in CMF-PBS. 0.8 ml of the cell suspension is added to 15 μg of plasmid DNA. Transfection is done by electroporation using a BIO RAD Gene Pulser (voltage = 1000 V; capacitor = 25 μF). After transfection cells are cultured in 15 ml Alpha+ medium with 10 % FCS for 3 days.

The dhfr-gene located on the pXMT3 plasmid allows selection for recombinant cells in a nucleoside depleted medium. 3 days after transfection cells are placed into Alpha-medium (MEM ALPHA MEDIUM without ribonucleosides and deoxyribonucleosides/Gibco) containing 10 % dialyzed fetal calf serum (FCSD). After 2 weeks of cultivation, recombinant cell colonies are visible. Cells are kept in Alpha- medium containing 10 % FCSD for 4 additional passages before gene amplification is started.

In the presence of methotrexate (MTX), dhfr and the genes linked to it are amplified, resulting in an increased expression of the transgene. Therefore, selection for recombinant cells in a nucleoside-depleted medium is followed by cultivation in the presence of 20 nM MTX in Alpha- medium containing 10 % FCSD. Further amplification is achieved by stepwise increases in methotrexate to 100 nM and 500 nM MTX.

Protein is produced by seeding pool T1/3-500 nM in Alpha- medium containing 10 % FCS (GIBCO) at a density of 9×10$^3$/cm$^2$ into roller bottles. The first supernatant is collected 5 days after seeding, followed by a switch to serum free Alpha- . The second harvest is collected 3 days later, yielding a total of I liter supernatant for purification.

A second batch is purified from 2 liter supernatant derived from pool T1/3-500 nM adapted to serum free growth conditions. Cells are seeded at 5×10$^4$ cells/ml and the supernatant collected 6 days after seeding.

## Example 8: Purification of fusion protein

The culture supernatants from Example 7 are purified by immunoaffinity chromatography on immobilized anti-FcεRI monoclonal antibodies (e.g. 5H5-F8 ; mouse 1gG1), produced and purified according to standard techniques:

a) Preparation of the chromatographic support:

The monoclonal antibodies are coupled to CNBr-activated Sepharose 4B (Pharmacia, Uppsala, Sweden) at a density of 10 mg antibodies / ml gel according to the manufacturer's instructions. Excess reactive groups are subsequently blocked with ethanolamine and the resin stored in PBS supplemented with 0.02 % NaN$_3$ until use.

b) Affinity chromatography:

Clear culture supernatant is applied to a 5 ml antibody column equilibrated in PBS at a flow rate of 0.5 ml/min. The absorbed material is eluted in 50 mM citric acid, 140 mM NaCl, pH 2.70. Protein containing fractions are immediately adjusted to pH 7.0 (NaOH) followed by sterile filtration.

c) Quantification / characterization:

The concentration of the dimeric fusion polypeptide is determined by absorption at 280 nm in its native conformation in 30 mM (3-[N-morpholino]propane)sulfonic acid (MOPS), pH 7.0 and in the denatured form (6 M guanidine.HCl). The corresponding molar absorption coefficient is calculated from the number of tryptophan, tyrosine and cystine residues using the tabulated absorption coefficients of these amino acids in model compounds and corrected for the difference in optical density between folded and unfolded protein. The fusion protein contains 17 tryptophans, 40 tyrosines and 21 cystines, which results in a theoretical extinction coefficient of 150840 $M^{-1}cm^{-1}$. The quality of the purified material is assessed by standard SDS-PAGE and by N-terminus automated gas-phase Edman degradation sequencing and mass spectrometry. Upon SDS-PAGE (**FIG. 15**) the polypeptide migrates with an apparent molecular weight of about 140 kDa, reflecting about 28 % glycosylation (theoretical MW without glycosylation: 108'863.61 Da).

## Claims

1.  A fusion polypeptide or a salt thereof of SEQ.ID.NO.3 or of residues $Val_{26}$-$Leu_{978}$ of SEQ.ID.NO.3.

2.  An isolated polynucleotide which is an intermediate in the preparation of a polypeptide or a salt thereof of claim 1 which comprises residues $Val_{28}$-$Leu_{978}$ of SEQ.ID.NO.3.

3.  A process for the preparation of a polypeptide or a salt thereof according to claim 1, which comprises

    (a) transforming a host cell with a vector comprising DNA encoding a polypeptide according to claim 1;
    (b) expressing the polypeptide in that cell to yield a fusion polypeptide as defined in claim 1; and
    (c) recovering the resultant polypeptide from the host cell, optionally in the form of a salt thereof.

4.  A vector comprising DNA encoding a polypeptide or a salt thereof according to claim 1.

5.  A transgenic non-human animal expressing a polypeptide or a salt thereof according to claim 1.

6.  A polypeptide according to claim 1 or a pharmaceutically acceptable salt thereof, for use as a medicament.

7.  A pharmaceutical composition comprising a polypeptide according to claim 1 or a pharmaceutically acceptable salt thereof, together with a pharmaceutically acceptable carrier or diluent.

8.  Use of a fusion polypeptide according to claim 1 in the preparation of a medicament for the treatment of IgE- or IgE-receptor mediated disorders.

## Patentansprüche

1.  Fusionspolypeptid oder ein Salz hiervon der SEQ.ID.NO.3 oder der Reste $Val_{26}$-$Leu_{978}$ der SEQ.ID.NO.3.

2.  Isoliertes Polynucleotid, das ein Zwischenprodukt bei der Herstellung eines Polypeptids oder eines Salzes hiervon nach Anspruch 1 ist, das die Reste $Val_{26}$-$Leu_{978}$ der SEQ.ID.NO.3 umfasst.

**3.** Verfahren zur Herstellung eines Polypeptids oder eines Salzes hiervon nach Anspruch 1, durch

(a) Transformation einer Wirtszelle mit einem Vektor, der DNA umfasst, die für ein Polypeptid nach Anspruch 1 kodiert,
(b) Expression des Polypeptids in dieser Zelle unter Erhalt eines Fusionspolypeptids gemäß der Definition in Anspruch 1, und
(c) Gewinnung des erhaltenen Polypeptids aus der Wirtszelle, optional in der Form eines Salzes ^ hiervon.

**4.** Vektor, der DNA umfasst, die für ein Polypeptid oder ein Salz hiervon nach Anspruch 1 kodiert.

**5.** Transgenes nicht humanes Tier, das ein Polypeptid oder ein Salz hiervon nach Anspruch 1 exprimiert.

**6.** Polypeptid nach Anspruch 1 oder ein pharmazeutisch akzeptables Salz hiervon, für die Verwendung als ein Arzneimittel.

**7.** Pharmazeutische Zusammensetzung, umfassend ein Polypeptid nach Anspruch 1 oder ein pharmazeutisch akzeptables Salz hiervon, zusammen mit einem pharmazeutisch akzeptablen Träger oder Verdünnungsmittel.

**8.** Verwendung eines Fusionspolypeptids nach Anspruch 1 zur Herstellung eines Arzneimittels für die Behandlung von Störungen, die durch IgE oder den IgE-Rezeptor mediiert werden.


**Revendications**

**1.** Polypeptide de fusion, ou l'un de ses sels de la SEQ ID N˚ 3, ou des résidus $Val_{26}$ - $Leu_{978}$ de la SEQ ID N˚ 3.

**2.** Polypeptide isolé qui est un intermédiaire dans la préparation d'un polypeptide, ou de l'un de ses sels selon la revendication 1, qui comprend les résidus $Val_{26}$ - $Leu_{978}$ de la SEQ ID N˚ 3.

**3.** Procédé de préparation d'un polypeptide, ou de l'un de ses sels selon la revendication 1, qui comprend :

(a) la transformation d'une cellule hôte avec un vecteur comprenant un ADN codant pour un polypeptide selon la revendication 1 ;
(b) l'expression du polypeptide dans cette cellule pour donner un polypeptide de fusion tel que défini dans la revendication 1 ; et
(c) la récupération du polypeptide résultant à partir de la cellule hôte, facultativement sous forme de l'un de ses sels.

**4.** Vecteur comprenant un ADN codant pour un polypeptide, ou l'un de ses sels selon la revendication 1.

**5.** Animal non humain transgénique exprimant un polypeptide, ou l'un de ses sels selon la revendication 1.

**6.** Polypeptide selon la revendication 1, ou l'un de ses sels pharmaceutiquement acceptables, à utiliser en tant que médicament.

**7.** Composition pharmaceutique comprenant un polypeptide selon la revendication 1, ou l'un de ses sels pharmaceutiquement acceptables, conjointement avec un support ou diluant pharmaceutiquement acceptable.

**8.** Utilisation d'un polypeptide de fusion selon la revendication 1 dans la préparation d'un médicament pour le traitement d'affections à médiation par l'IgE ou un récepteur d'IgE.

## Figure 1

Serum half life in mice

A

## Figure 1 (cont.)

B

## Figure 1 (cont.)

C

## Figure 2

Passive cutaneous anaphylaxis reaction

## Figure 3

Schematic representation of three fusion polypeptides

I. Monomers:

   1. HSA-IgE$^R$        (HSA-<u>LG</u>GGG<u>GS</u>-IgE$^R$)
                                  |     |
                              MstII  BamHI

   2. IgE$^R$-HSA        (IgE$^R$-<u>AS</u>GGGG<u>GS</u>-HSA)
                                |       |
                             NheI    BamHI

II. Dimer:

   1. IgE$^R$-HSA-IgE$^R$     (IgE$^R$-<u>AS</u>GGGG<u>GS</u>-HSA-<u>LG</u>GGG<u>GS</u>-IgE$^R$)
                               |      |      |      |
                            NheI   BamHI  MstII  BamHI

## Figure 4

PCR primers

# IgE RECEPTOR cDNA

| signal | extracellular domain | TM cytoplasmic |
|--------|---------------------|----------------|

20     31

18     19 **

TA Clone

A. HSA-leading:     pEK1

18     19 **

B. IgER-leading     IgER/TA#1

20     31

C. IgER alone     IgER FL/TA #34

20     19 **

**Figure 5**

PCR primers

EcoRI

Ncol

Spel

MstII EcoRI

HindIII

prepro    HSA/SK #17

24                                                    25 **

SK clone

A. HSA leading                                pEK7

24                                                    28
                                                        29

B. IgER leading                              HSA/SK#5

26         27

C. HSA alone                                 HSA/SK #17

24                                                    25 **

## Figure 6

HSA sequencing oligonucleotides

**HSA sequencing oligos:**

#1   s/n459031-sequencing oligo for HSA @ ~200bp-coding strand
          5'TCAAAGCCTTGGTGTTGATTG3'

#2   s/n458896-sequencing oligo for HSA @ ~400bp-coding strand
          5'TGAAATGGCTGACTGCTGTG3'

#3   s/n458858-sequencing oligo for HSA @ ~600bp-coding strand
          5'AGGTATAAAGCTGCTTTTACAG3'

#4   s/n450959-sequencing oligo for HSA @ ~800bp-coding strand
          5'TGAGCCAGAGATTTCCCAAAG3'

#5   s/n451995-sequencing oligo for HSA @ ~1000bp-coding strand
          5'TCCCACTGCATTGCCGAAGTG3'

#6   s/n485788-sequencing oligo for HSA @ ~1200bp-coding strand
          5'CTAGAGAAGTGCTGTGCCGCT3'

#7   s/n480661-sequencing oligo for HSA @ ~1400bp-coding strand
          5'TGTCAACTCCAACTCTTGT3'

#8   s/n451389-sequencing oligo for HSA @ ~1600bp-coding strand
          5'CAGCTCTGGAAGTCGATGAAA3'

#9   s/n462782-sequencing oligo for HSA @ ~780bp-noncoding strand
          5'CTTTGAAAGCTCTTTCTCCA3'

#10  s/n437503-sequencing oligo for HSA @ ~1335bp-noncoding strand
          5'ATTCTGGAATTTGTACTCTCC3'

#12  s/n434978-sequencing oligo near 5' end of HSA for sequencing linkers-noncoding
          strand
          5'ACACTGCTGAAGATACTGAGC3'

#16  s/n465147-sequencing oligo for HSA @ ~550bp-noncoding strand
          5'TCTGGCAATTTCATATAAGTA3'

#17  s/n428935-sequencing oligo for HSA @ ~1455bp-coding strand
          5'ATGTTGTAAACATCCTGAAGC3'

#22  s/n466444-sequencing oligo near 3' end of HSA for sequencing linkers-coding strand
          5'ACCTGCTTTGCCGAGGAGGGT3'

#30  Sequencing oligo for HSA @ ~150bp-coding strand
          5'ACAAGAGTGAGGTTGCTCATC3'

## Figure 7

IgE^R sequencing oligonucleotides

#11   s/n479818-sequencing oligo near 5' end of IgE^R for sequencing linkers-noncoding strand

    5'CCTTTAAATATTCTATTCCAT3'

#13   s/n486284-sequencing oligo for IgE^R @ 220bp-coding strand
    5'GAAGTCAGTTCCACCAAATGGT3'

#23   s/n418915-sequencing oligo for IgE^R @ 420bp-coding strand
    5'GATGGAGGGCCAGCCCCTCTT3'

## Figure 8

(A) Mutagenic oligonucleotides for HSA

#14    s/n450055-mutagenic oligo for HSA to change E back to K-positive
            5'TGTGAGCTTTTTAAGCAGCTTGGAG3'

#15    s/n464834-mutagenic oligo for HSA to change E back to K-negative control for
complementary strand
            5'CTCCAAGCTGCTTAAAAAGCTCACA3'

## Figure 8 (cont.)

### (B) PCR and linker oligonucleotides

#18    s/n407231-PCR oligo for IgE$^R$ for HSA-IgE$^R$ construct at 5' end adding a BamHI site
for cloning-coding strand

        5'TCAT<u>GGATCC</u>GTCCCTCAGAAACCTAAGGTCTCCTTGAAC3'
               BamHI

#19    s/n481113-PCR oligo for IgE$^R$ for HSA-IgE$^R$ construct at 3' end of extracellular
domain adding stop, EcoRI and SalI-non-coding strand

        5'TCAT<u>GTCGAC</u> <u>GAATTC</u> TTACTATAGCCAGTACTTCTCACGCGGAGC
          SalI      EcoRI    *     *
        TTTTAT3'

#20    s/n432172-PCR oligo for IgE$^R$ for IgE$^R$-HSA construct at 5' end adding SstI, EcoRI,
and Kozak-coding strand

        5'TCAT <u>GAGCTC</u> <u>GAATTC</u> <u>ACC</u>ATGGCTCCTGCCATGGAATCCCCTACT
          SstI      EcoRI   Kozak
        CTA3'

#24    s/n489617-PCR oligo for HSA for HSA-IgE$^R$ construct at 5' end adding SpeI, EcoRI
and Kozak-coding strand

        5'TCAT <u>ACTAGT</u> <u>GAATTC</u> <u>ACC</u> ATGAAGTGGGTAACCTTTATTTCCCTT
           SpeI     EcoRI   Kozak
        CTT3'

#25    s/n412766-PCR oligo for HSA for HSA-IgE$^R$ construct at 3' end adding stop, EcoRI
and HindIII--non-coding strand

        5'TCAT <u>AAGCTT</u> <u>GAATTC</u> CTATTATAAG<u>CCTAAGG</u>CAGCTTGACTTGC
          HindIII  EcoRI  *    *    MstII
        AGC3'

**B**

## Figure 8 (cont.)

#26 PCR oligo for HSA for IgE^R-HSA construct at 5' end adding NotI, NheI, linker, and BamHI-coding strand

<pre>
     5'GCGGCCGC GCTAGCGGTGGAGGTGGATCCGATGCAC
        NotI       NheI            BamHI
        ACAAGAGTGAGGTTGCTCATCGGTTT3'
</pre>

#27 PCR oligo for HSA for IgE^R-HSA construct at NcoI site of HSA-non-coding strand

<pre>
     5'TCATCCATGGCAGCATTCCGTGTGGACTTTGGTAAGA3'
          NcoI
</pre>

#28 Linker oligo for HSA-IgE^R construct to be ligated as a MstII, HindIII fragment-coding strand

<pre>
     5'TTAGGTGGAGGTGGATCCA3'
        MstII        BamHI
</pre>

#29 Linker oligo for HSA-IgE^R construct to be ligated as a MstII, HindIII fragment-non-coding strand

<pre>
     5'AGCTT GGATCCACCTCCACC3'
        HindIII BamHI
</pre>

#31 PCR oligo for IgE^R for IgE^R-HSA construct at 3' end of extracellular domain adding NotI and NheI-deletes a second NheI site-non-coding strand

<pre>
     5'TCATGCGGCCGC GCTAGCAAGCCAGTACTTCTCACGCGGAGCTTTT ?
          NotI         NheI

     A3'
</pre>

**B** (continued)

## Figure 9

Construction of vector HSA-IGER/SK#49

**Figure 10**

Construction of vector IgER-HSA/SK#1

### Figure 11

Construction of vectors HSA-IgER Pst Sal/SK#37 and R-H-R/SK #50

**Figure 12**

Nucleotide and amino acid sequence of HSA ·

```
ATGAAGTGGGTAACCTTTATTTCCCTTCTTTTTCTCTTTAGCTCGGCTTATTCCAGGGGT
M  K  W  V  T  F  I  S  L  L  F  L  F  S  S  A  Y  S  R  G

GTGTTTCGTCGAGATGCACACAAGAGTGAGGTTGCTCATCGGTTTAAAGATTTGGGAGAA
V  F  R  R  D  A  H  K  S  E  V  A  H  R  F  K  D  L  G  E
   GAAAATTTCAAAGCCTTGGTGTTGATTGCCTTTGCTCAGTATCTTCAGCAGTGTCCATTT
E  N  F  K  A  L  V  L  I  A  F  A  Q  Y  L  Q  Q  C  P  F
   GAAGATCATGTAAAATTAGTGAATGAAGTAACTGAATTTGCAAAAACATGTGTAGCTGAT
E  D  H  V  K  L  V  N  E  V  T  E  F  A  K  T  C  V  A  D
   GAGTCAGCTGAAAATTGTGACAAATCACTTCATACCCTTTTTGGAGACAAATTATGCACA
E  S  A  E  N  C  D  K  S  L  H  T  L  F  G  D  K  L  C  T
   GTTGCAACTCTTCGTGAAACCTATGGTGAAATGGCTGACTGCTGTGCAAAACAAGAACCT
V  A  T  L  R  E  T  Y  G  E  M  A  D  C  C  A  K  Q  E  P
   GAGAGAAATGAATGCTTCTTGCAACACAAAGATGACAACCCAAACCTCCCCCGATTGGTG
E  R  N  E  C  F  L  Q  H  K  D  D  N  P  N  L  P  R  L  V
   AGACCAGAGGTTGATGTGATGTGCACTGCTTTTCATGACAATGAAGAGACATTTTTGAAA
R  P  E  V  D  V  M  C  T  A  F  H  D  N  E  E  T  F  L  K
   AAATACTTATATGAAATTGCCAGAAGACATCCTTACTTTTATGCCCCGGAACTCCTTTTC
K  Y  L  Y  E  I  A  R  R  H  P  Y  F  Y  A  P  E  L  L  F
   TTTGCTAAAAGGTATAAAGCTGCTTTTACAGAATGTTGCCAAGCTGCTGATAAAGCTGCC
F  A  K  R  Y  K  A  A  F  T  E  C  C  Q  A  A  D  K  A  A
   TGCCTGTTGCCAAAGCTCGATGAACTTCGGGATGAAGGGAAGGCTTCGTCTGCCAAACAG
C  L  L  P  K  L  D  E  L  R  D  E  G  K  A  S  S  A  K  Q
   AGACTCAAATGTGCCAGTCTCCAAAAATTTGGAGAAAGAGCTTTCAAAGCATGGGCAGTG
R  L  K  C  A  S  L  Q  K  F  G  E  R  A  F  K  A  W  A  V.
   GCTCGCCTGAGCCAGAGATTTCCCAAAGCTGAGTTTGCAGAAGTTTCCAAGTTAGTGACA
A  R  L  S  Q  R  F  P  K  A  E  F  A  E  V  S  K  L  V  T
   GATCTTACCAAAGTCCACACGGAATGCTGCCATGGAGATCTGCTTGAATGTGCTGATGAC
D  L  T  K  V  H  T  E  C  C  H  G  D  L  L  E  C  A  D  D
   AGGGCGGACCTTGCCAAGTATATCTGTGAAAATCAGGATTCGATCTCCAGTAAACTGAAG
R  A  D  L  A  K  Y  I  C  E  N  Q  D  S  I  S  S  K  L  K
   GAATGCTGTGAAAAACCTCTGTTGGAAAAATCCCACTGCATTGCCGAAGTGGAAAATGAT
E  C  C  E  K  P  L  L  E  K  S  H  C  I  A  E  V  E  N  D
   GAGATGCCTGCTGACTTGCCTTCATTAGCTGCTGATTTTGTTGAAAGTAAGGATGTTTGC
E  M  P  A  D  L  P  S  L  A  A  D  F  V  E  S  K  D  V  C
   AAAAACTATGCTGAGGCAAAGGATGTCTTCCTGGGCATGTTTTTGTATGAATATGCAAGA
K  N  Y  A  E  A  K  D  V  F  L  G  M  F  L  Y  E  Y  A  R
```

## Figure 12 (cont.)

```
    AGGCATCCTGATTACTCTGTCGTGCTGCTGCTGAGACTTGCCAAGACATATGAAACCACT
R H P D Y S V V L L L R L A K T Y E T T
    CTAGAGAAGTGCTGTGCCGCTGCAGATCCTCATGAATGCTATGCCAAAGTGTTCGATGAA
L E K C C A A A D P H E C Y A K V F D E
    TTTAAACCTCTTGTGGAAGAGCCTCAGAATTTAATCAAACAAAACTGTGAGCTTTTTAAG
F K P L V E E P Q N L I K Q N C E L F K
    CAGCTTGGAGAGTACAAATTCCAGAATGCGCTATTAGTTCGTTACACCAAGAAAGTACCC
Q L G E Y K F Q N A L L V R Y T K K V P
    CAAGTGTCAACTCCAACTCTTGTAGAGGTCTCAAGAAACCTAGGAAAAGTGGGCAGCAAA
Q V S T P T L V E V S R N L G K V G S K
    TGTTGTAAACATCCTGAAGCAAAAAGAATGCCCTGTGCAGAAGACTATCTATCCGTGGTC
C C K H P E A K R M P C A E D Y L S V V
    CTGAACCAGTTATGTGTGTTGCATGAGAAAACGCCAGTAAGTGACAGAGTCACAAAATGC


L N Q L C V L H E K T P V S D R V T K C
    TGCACAGAGTCCTTGGTGAACAGGCGACCATGCTTTTCAGCTCTGGAAGTCGATGAAACA
C T E S L V N R R P C F S A L E V D E T
    TACGTTCCCAAAGAGTTTAATGCTGAAACATTCACCTTCCATGCAGATATATGCACACTT
Y V P K E F N A E T F T F H A D I C T L
    TCTGAGAAGGAGAGACAAATCAAGAAACAAACTGCACTTGTTGAGCTTGTGAAACACAAG
S E K E R Q I K K Q T A L V E L V K H K
    CCCAAGGCAACAAAAGAGCAACTGAAAGCTGTTATGGATGATTTCGCAGCTTTTGTAGAG
P K A T K E Q L K A V M D D F A A F V E

AAGTGCTGCAAGGCTGACGATAAGGAGACCTGCTTTGCCGAGGAGGGTAAAAAACTTGTT
K C C K A D D K E T C F A E E G K K L V

GCTGCAAGTCAAGCTGCCTTAGGCTTA
A A S Q A A L G L
```

**Figure 13**

Nucleotide and amino acid sequence of IgE[R]

ATGGCTCCTGCCATGGAATCCCCTACTCTACTGTGTGTAGCCTTACTGTTCTTCGCTCCA
M A P A M E S P T L L C V A L L F F A P -
GATGGCGTGTTAGCAGTCCCTCAGAAACCTAAGGTCTCCTTGAACCCTCCATGGAATAGA
D G V L A V P Q K P K V S L N P P W N R -
ATATTTAAAGGAGAGAATGTGACTCTTACATGTAATGGGAACAATTTCTTTGAAGTCAGT
I F K G E N V T L T C N G N N F F E V S -
TCCACCAAATGGTTCCACAATGGCAGCCTTTCAGAAGAGACAAATTCAAGTTTGAATATT
S T K W F H N G S L S E E T N S S L N I -
GTGAATGCCAAATTTGAAGACAGTGGAGAATACAAATGTCAGCACCAACAAGTTAATGAG
V N A K F E D S G E Y K C Q H Q Q V N E -
AGTGAACCTGTGTACCTGGAAGTCTTCAGTGACTGGCTGCTCCTTCAGGCCTCTGCTGAG
S E P V Y L E V F S D W L L L Q A S A E

GTGGTGATGGAGGGCCAGCCCCTCTTCCTCAGGTGCCATGGTTGGAGGAACTGGGATGTG
V V M E G Q P L F L R C H G W R N W D V
TACAAGGTGATCTATTATAAGGATGGTGAAGCTCTCAAGTACTGGTATGAGAACCACAAC
Y K V I Y Y K D G E A L K Y W Y E N H N
ATCTCCATTACAAATGCCACAGTTGAAGACAGTGGAACCTACTACTGTACGGGCAAAGTG
I S I T N A T V E D S G T Y Y C T G K V
TGGCAGCTGGACTATGAGTCTGAGCCCCTCAACATTACTGTAATAAAAGCTCCGCGTGAG
W Q L D Y E S E P L N I T V I K A P R E
AAGTACTGGCTACAATTTTTTATCCCATTGTTGGTGGTGATTCTGTTTGCTGTGGACACA
K Y W L Q F F I P L L V V I L F A V D T
GGATTATTTATCTCAACTCAGCAGCAGGTCACATTTCTCTTGAAGATTAAGAGAACCAGG
G L F I S T Q Q Q V T F L L K I K R T R

AAAGGCTTCAGACTTCTGAACCCACATCCTAAGCCAAACCCCAAAAACAACTG
K G F R L L N P H P K P N P K N N

## Figure 14

Nucleotide and amino acid sequence of the EcoRI fragment of R-H-R/SK #50

```
GAATTCACCATGGCTCCTGCCATGGAATCCCCTACTCTACTGTGTGTAGCCTTACTGTTC
     M  A  P  A  M  E  S  P  T  L  L  C  V  A  L  L  F
     TTCGCTCCAGATGGCGTGTTAGCAGTCCCTCAGAAACCTAAGGTCTCCTTGAACCCTCCA
 F  A  P  D  G  V  L  A  V  P  Q  K  P  K  V  S  L  N  P  P
     TGGAATAGAATATTTAAAGGAGAGAATGTGACTCTTACATGTAATGGGAACAATTTCTTT
 W  N  R  I  F  K  G  E  N  V  T  L  T  C  N  G  N  N  F  F
     GAAGTCAGTTCCACCAAATGGTTCCACAATGGCAGCCTTTCAGAAGAGACAAATTCAAGT
 E  V  S  S  T  K  W  F  H  N  G  S  L  S  E  E  T  N  S  S
     TTGAATATTGTGAATGCCAAATTTGAAGACAGTGGAGAATACAAATGTCAGCACCAACAA
 L  N  I  V  N  A  K  F  E  D  S  G  E  Y  K  C  Q  H  Q  Q
     GTTAATGAGAGTGAACCTGTGTACCTGGAAGTCTTCAGTGACTGGCTGCTCCTTCAGGCC
 V  N  E  S  E  P  V  Y  L  E  V  F  S  D  W  L  L  L  Q  A

TCTGCTGAGGTGGTGATGGAGGGCCAGCCCCTCTTCCTCAGGTGCCATGGTTGGAGGAAC
 S  A  E  V  V  M  E  G  Q  P  L  F  L  R  C  H  G  W  R  N
     TGGGATGTGTACAAGGTGATCTATTATAAGGATGGTGAAGCTCTCAAGTACTGGTATGAG
 W  D  V  Y  K  V  I  Y  Y  K  D  G  E  A  L  K  Y  W  Y  E
     AACCACAACATCTCCATTACAAATGCCACAGTTGAAGACAGTGGAACCTACTACTGTACG
 N  H  N  I  S  I  T  N  A  T  V  E  D  S  G  T  Y  Y  C  T
     GGCAAAGTGTGGCAGCTGGACTATGAGTCTGAGCCCCTCAACATTACTGTAATAAAAGCT
 G  K  V  W  Q  L  D  Y  E  S  E  P  L  N  I  T  V  I  K  A
     CCGCGTGAGAAGTACTGGCTTgctagcggtggaggtggatccGATGCACACAAGAGTGAG
 P  R  E  K  Y  W  L  A  S  G  G  G  G  S  D  A  H  K  S  E
     GTTGCTCATCGGTTTAAAGATTTGGGAGAAGAAAATTTCAAAGCCTTGGTGTTGATTGCC
 V  A  H  R  F  K  D  L  G  E  E  N  F  K  A  L  V  L  I  A
     TTTGCTCAGTATCTTCAGCAGTGTCCATTTGAAGATCATGTAAAATTAGTGAATGAAGTA
 F  A  Q  Y  L  Q  Q  C  P  F  E  D  H  V  K  L  V  N  E  V
     ACTGAATTTGCAAAAACATGTGT{GCTGATGAGTCAGCTGAAAATTGTGACAAATCACTT
 T  E  F  A  K  T  C  V  A  D  E  S  A  E  N  C  D  K  S  L
```

## Figure 14 (cont.)

```
CATACCCTTTTTGGAGACAAATTATGCACAGTTGCAACTCTTCGTGAAACCTATGGTGAA
 H  T  L  F  G  D  K  L  C  T  V  A  T  L  R  E  T  Y  G  E
  ATGGCTGACTGCTGTGCAAAACAAGAACCTGAGAGAAATGAATGCTTCTTGCAACACAAA
 M  A  D  C  C  A  K  Q  E  P  E  R  N  E  C  F  L  Q  H  K
  GATGACAACCCAAACCTCCCCCGATTGGTGAGACCAGAGGTTGATGTGATGTGCACTGCT
 D  D  N  P  N  L  P  R  L  V  R  P  E  V  D  V  M  C  T  A
  TTTCATGACAATGAAGAGACATTTTTTGAAAAAATACTTATATGAAATTGCCAGAAGACAT
 F  H  D  N  E  E  T  F  L  K  K  Y  L  Y  E  I  A  R  R  H
  CCTTACTTTTATGCCCCGGAACTCCTTTTCTTTGCTAAAAGGTATAAAGCTGCTTTTACA
 P  Y  F  Y  A  P  E  L  L  F  F  A  K  R  Y  K  A  A  F  T
  GAATGTTGCCAAGCTGCTGATAAAGCTGCCTGCCTGTTGCCAAAGCTCGATGAACTTCGG
 E  C  C  Q  A  A  D  K  A  A  C  L  L  P  K  L  D  E  L  R
  GATGAAGGGAAGGCTTCGTCTGCCAAACAGAGACTCAAgTGTGCCAGTCTCCAAAAATTT
 D  E  G  K  A  S  S  A  K  Q  R  L  K  C  A  S  L  Q  K  F
  GGAGAAAGAGCTTTCAAAGCATGGGCAGTaGCTCGCCTGAGCCAGAGATTTCCCAAAGCT
 G  E  R  A  F  K  A  W  A  V  A  R  L  S  Q  R  F  P  K  A
  GAGTTTGCAGAAGTTTCCAAGTTAGTGACAGATCTTACCAAAGTCCACACGGAATGCTGC
 E  F  A  E  V  S  K  L  V  T  D  L  T  K  V  H  T  E  C  C
  CATGGAGATCTGCTTGAATGTGCTGATGACAGGGCGGACCTTGCCAAGTATATCTGTGAA
 H  G  D  L  L  E  C  A  D  D  R  A  D  L  A  K  Y  I  C  E
  AATCAaGATTCGATCTCCAGTAAACTGAAGGAATGCTGTGAAAAACCTCTGTTGGAAAAA
 N  Q  D  S  I  S  S  K  L  K  E  C  C  E  K  P  L  L  E  K
  TCCCACTGCATTGCCGAAGTGGAAAATGATGAGATGCCTGCTGACTTGCCTTCATTAGCT
 S  H  C  I  A  E  V  E  N  D  E  M  P  A  D  L  P  S  L  A
  GCTGATTTTGTTGAAAGTAAGGATGTTTGCAAAAACTATGCTGAGGCAAAGGATGTCTTC
 A  D  F  V  E  S  K  D  V  C  K  N  Y  A  E  A  K  D  V  F
  CTGGGCATGTTTTTGTATGAATATGCAAGAAGGCATCCTGATTACTCTGTCGTGCTGCTG
 L  G  M  F  L  Y  E  Y  A  R  R  H  P  D  Y  S  V  V  L  L
  CTGAGACTTGCCAAGACATATGAAACCACTCTAGAGAAGTGCTGTGCCGCTGCAGATCCT
 L  R  L  A  K  T  Y  E  T  T  L  E  K  C  C  A  A  A  D  P
  CATGAATGCTATGCCAAAGTGTTCGATGAATTTAAACCTCTTGTGGAAGAGCCTCAGAAT
 H  E  C  Y  A  K  V  F  D  E  F  K  P  L  V  E  E  P  Q  N
  TTAATCAAACAAAAtTGTGAGCTTTTTAAGCAGCTTGGAGAGTACAAATTCCAGAATGCG
 L  I  K  Q  N  C  E  L  F  K  Q  L  G  E  Y  K  F  Q  N  A
  CTATTAGTTCGTTACACCAAGAAAGTACCCCAAGTGTCAACTCCAACTCTTGTAGAGGTC
 L  L  V  R  Y  T  K  K  V  P  Q  V  S  T  P  T  L  V  E  V
  TCAAGAAACCTAGGAAAAGTGGGCAGCAAATGTTGTAAACATCCTGAAGCAAAAAGAATG
 S  R  N  L  G  K  V  G  S  K  C  C  K  H  P  E  A  K  R  M
  CCCTGTGCAGAAGACTATCTATCCGTGGTCCTGAACCAGTTATGTGTGTTGCATGAGAAA
 P  C  A  E  D  Y  L  S  V  V  L  N  Q  L  C  V  L  H  E  K
```

## Figure 14 (cont.)

```
ACGCCAGTAAGTGACAGAGTCACcAAATGCTGCACAGAaTCCTTGGTGAACAGGCGACCA
 T  P  V  S  D  R  V  T  K  C  C  T  E  S  L  V  N  R  R  P
    TGCTTTTCAGCTCTGGAAGTCGATGAAACATACGTTCCCAAAGAGTTTAATGCTGAAACA
 C  F  S  A  L  E  V  D  E  T  Y  V  P  K  E  F  N  A  E  T
    TTCACCTTCCATGCAGATATATGCACACTTTCTGAGAAGGAGAGACAAATCAAGAAACAA
 F  T  F  H  A  D  I  C  T  L  S  E  K  E  R  Q  I  K  K  Q
    ACTGCACTTGTTGAGCTTGTGAAACACAAGCCCAAGGCAACAAAAGAGCAACTGAAAGCT
 T  A  L  V  E  L  V  K  H  K  P  K  A  T  K  E  Q  L  K  A
    GTTATGGATGATTTCGCAGCTTTTTGTAGAGAAGTGCTGCAAGGCTGACGATAAGGAGACC
 V  M  D  D  F  A  A  F  V  E  K  C  C  K  A  D  D  K  E  T
    TGCTTTGCCGAGGAGGGTAAAAAACTTGTTGCTGCAAGTCAAGCTGCCTTAGGtggaggt

 C  F  A  E  E  G  K  K  L  V  A  A  S  Q  A  A  L  G  G  G
    ggatccGTCCCTCAGAAACCTAAGGTCTCCTTGAACCCTCCATGGAATAGAATATTTAAA
 G  S  V  P  Q  K  P  K  V  S  L  N  P  P  W  N  R  I  F  K
    GGAGAGAATGTGACTCTTACATGTAATGGGAACAATTTCTTTGAAGTCAGTTCCACCAAA
 G  E  N  V  T  L  T  C  N  G  N  N  F  F  E  V  S  S  T  K
    TGGTTCCACAATGGCAGCCTTTCAGAAGAGACAAATTCAAGTTTGAATATTGTGAATGCC
 W  F  H  N  G  S  L  S  E  E  T  N  S  S  L  N  I  V  N  A
    AAATTTGAAGACAGTGGAGAATACAAATGTCAGCACCAACAAGTTAATGAGAGTGAACCT
 K  F  E  D  S  G  E  Y  K  C  Q  H  Q  Q  V  N  E  S  E  P
    GTGTACCTGGAAGTCTTCAGTGACTGGCTGCTCCTTCAGGCCTCTGCTGAGGTGGTGATG
 V  Y  L  E  V  F  S  D  W  L  L  L  Q  A  S  A  E  V  V  M

GAGGGCCAGCCCCTCTTCCTCAGGTGCCATGGTTGGAGGAACTGGGATGTGTACAAGGTG
 E  G  Q  P  L  F  L  R  C  H  G  W  R  N  W  D  V  Y  K  V
    ATCTATTATAAGGATGGTGAAGCTCTCAAGTACTGGTATGAGAACCACAACATCTCCATT
 I  Y  Y  K  D  G  E  A  L  K  Y  W  Y  E  N  H  N  I  S  I
    ACAAATGCCACAGTTGAAGACAGTGGAACCTACTACTGTACGGGCAAAGTGTGGCAGCTG
 T  N  A  T  V  E  D  S  G  T  Y  Y  C  T  G  K  V  W  Q  L
    GACTATGAGTCTGAGCCCCTCAACATTACTGTAATAAAAGCTCCGCGTGAGAAGTACTGG
 D  Y  E  S  E  P  L  N  I  T  V  I  K  A  P  R  E  K  Y  W

CTATAGTAAGAATTC
 L  *  *
```

## Figure 15

SDS-PAGE of purified mature fusion polypeptide of Example 7

# EP 0 917 581 B1

## REFERENCES CITED IN THE DESCRIPTION

### Patent documents cited in the description

- US 4962035 A, Leder
- WO 9315199 A
- EP 648499 A
- EP 413622 A
- US 5487993 A
- US 4683195 A
- US 4683202 A
- US 08690216 B

### Non-patent literature cited in the description

- **M. CAPRON ; A.CAPRON.** *Science,* vol. 264
- **G-C. MUDDE et al.** *Allergy,* 1995, vol. 50, 193-199
- **J. HAKIMI et al.** *J. Biol. Chem.,* vol. 265 (19901), 22079-22081
- **U. BLANK et al.** *J. Biol Chem.,* 1991, vol. 266, 2639-2646
- **D. DOMBROWICZ et al.** *Cell,* 1993, vol. 75, 969-976
- **C. RA et al.** *Int.Immunol,* 1993, vol. 5, 47-54
- **M. HAAK-FRENDSCHO et al.** *Immunol,* 1993, vol. 151, 351-358
- **Y. YANAGIHARA et al.** *J. Clin. Invest,* 1994, vol. 94, 2162-2165
- **J.KOCHAN et al.** *Nucleic Acids Research,* 1988, vol. 16, 3584
- **F.F. CLERC et al.** *J.Chromatogr,* 1994, vol. 662, 245-259
- **T.A. WALDMANN.** Albumin Structure. Function and Uses. Pergamon Press, 1977, 255-275
- **H. LU et al.** *FEBS Lett,* 1994, vol. 356, 56-59
- **P. YEH et al.** *PNAS USA,* 1992, vol. 89, 1904-1908
- **D.C. CARTER ; JX. HO.** *Adv. Prot. Chem.,* 1994, vol. 45, 153-203
- **A. DUGAICZYK et al.** *PNAS USA,* 1982, vol. 79, 71-75
- Molecular Cloning: A Laboratory Manual. Cold Spring Harbor Laboratory Press, 1989
- **A. SHIMIZU et al.** *Proc.Nat.Acad.Sci.USA,* 1988, vol. 85, 1907-1911
- The Polymerase Chain Reaction: Getting Started. **C.R.M. BANGHAM.** Protocols in Human Molecular Genetics. Human Press, 1991, 1-8
- **M.W. ROBERTSON.** *J. Biol. Chem.,* 1993, vol. 268, 12736-12743
- **A. SHIMIZU et al.** *PNAS USA,* 1988, vol. 85, 1907-1911
- **C.F. GOOCHEE et al.** *BioTechnol,* 1991, vol. 9, 1347-1355
- **R.J. KAUFMAN et al.** *EMBO J,* 1987, vol. 6, 187-193
- **J. SAMBROOK et al.** *GENETIC ENGINEERING: PRINCIPLES AND METHODS,* 1989
- **R.J. KAUFMAN.** Genetic Engineering: Principles and Methods. Plenum Press, 1987, vol. 9, 156-198
- **M.-A. MORREN et al.** *J. Am. Acad. Dermatol,* 1994, vol. 31, 467-473
- **H. GRANLUND et al.** *Br. J. Dermatol.,* 1995, vol. 132, 106-112
- **D.R. HURWITZ et al.** *Transgenic Research,* 1994, vol. 3, 365-375
- Polymerase Chain Reaction. Cold Spring Harbor Laboratory Press, 1989
- **T.A. KUNKEL.** *Proc.Nat.Acad.Sci.USA,* 1985, vol. 82, 488-492